(19) Europäisches Patentamt
European Patent Office
Office européen des brevets

(11) Publication number: 0 097 961 B1

# (12) EUROPEAN PATENT SPECIFICATION

(45) Date of publication of patent specification: 11.11.87

(51) Int. Cl.$^4$: C 07 D 501/46, A 61 K 31/545

(21) Application number: 83106304.5

(22) Date of filing: 28.06.83

## (54) Cephalosporin derivatives, a process for the manufacture thereof and pharmaceutical compositions containing said derivatives.

(30) Priority: 28.06.82 US 392866

(43) Date of publication of application:
11.01.84 Bulletin 84/02

(45) Publication of the grant of the patent:
11.11.87 Bulletin 87/46

(84) Designated Contracting States:
AT BE CH DE FR GB IT LI LU NL SE

(58) References cited:
EP-A-0 064 740
EP-A-0 074 268
FR-A-2 137 899
FR-A-2 348 218
FR-A-2 385 722

The file contains technical information submitted after the application was filed and not included in this specification

(73) Proprietor: Bristol-Myers Company
345 Park Avenue
New York New York 10154 (US)

(72) Inventor: Okumura, Jun
1-21-2-101, Utsukushigaoka
Midori-ku Yokohama (JP)
Inventor: Imae, Kiyoto
46-1-812, Suenaga
Katasu-ku Kawasaki (JP)
Inventor: Aburaki, Shimpei
7-2-17-305 Todoroki Setagaya-ku
Tokyo (JP)
Inventor: Naito, Takayuki
2657-45, Ohzenji
Asao-ku Kawasaki (JP)
Inventor: Narita, Yukio
634-4-411, Noba-cho
Konan-ku Yokohama (JP)

(74) Representative: Kinzebach, Werner, Dr.
Patentanwälte Reitstötter, Kinzebach und Partner Sternwartstrasse 4 Postfach 86 06 49
D-8000 München 86 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 097 961 B1

Courier Press, Leamington Spa, England.

**Description**

This invention relates to novel cephalosporin derivatives of the formula

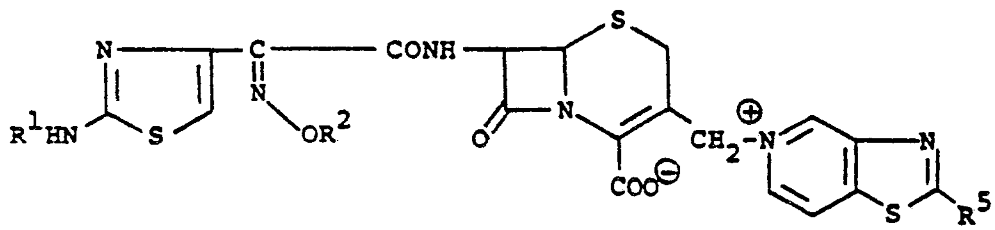

wherein $R^1$ is hydrogen or a conventional amino-protecting group, $R^2$ is a straight or branched chain alkyl group containing from 1 to 4 carbon atoms, allyl, 2-butenyl, 3-butenyl or a group of the formula

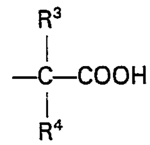

in which $R^3$ and $R^4$ each are independently hydrogen, methyl or ethyl, or $R^3$ and $R^4$, taken together with the carbon atom to which they are attached, may be a cycloalkylidene ring containing from 3 to 5 carbon atoms, and $R^5$ is hydrogen, amino, $C_1$—$C_6$-alkyl, $C_1$—$C_6$-alkoxy or $C_1$—$C_6$-alkylthio, and nontoxic pharmaceutically acceptable salts, physiologically hydrolyzable esters and solvates thereof. Processes for their preparation also are described.

U.K. Patent Specification No. 1,399,086 contains a generic disclosure encompassing a vast number of cephalosporins of the formula

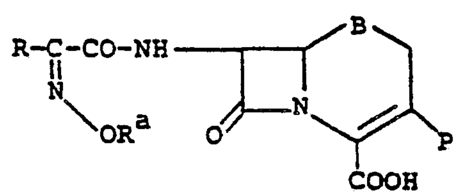

wherein R is hydrogen or an organic group, $R^a$ is an etherifying monovalent organic group linked to the oxygen through a carbon atom, B is >S or >S→O, and P is an organic group. However, the 2-aminothiazol-4-yl group is not identified as a possible R substituent. Although P may be the group —$CH_2$Y in which Y may be the residue of a nitrogen-containing heterocycle, only pyridinium and 4-carbamoylpyridinium are exemplified, and there is no suggestion that Y may be the 5-thiazolo[4,5-c]pyridinio moiety. U.S. Patent 3,971,778 and its divisionals Nos. 4,024,133, 4,024,137, 4,064,346, 4,033,950, 4,079,178, 4,091,209, 4,092,477 and 4,093,803 have similar disclosures.

U.S. 4,278,793 contains a generic disclosure encompassing a vast number of cephalosporin derivatives of the formula

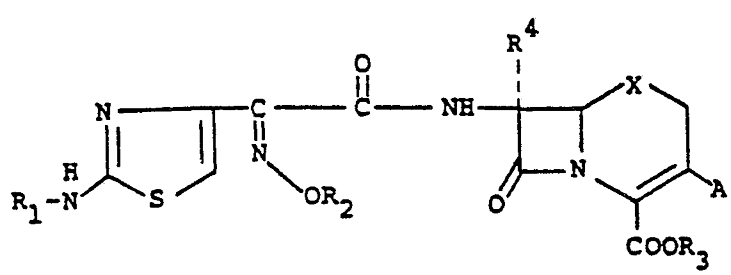

in which the variables $R_1$, $R_2$, $R_3$, $R_4$, X and A include generic definitions of the corresponding substituents of the compounds of Formula I claimed herein. Substituent A may be the group —$CH_2$Y in which Y may be the residue of a nucleophilic group (the only heterocyclic rings disclosed for Y are pyridinium, quinolinium and isoquinolinium). In the 20 columns of definitions of the various substituent groups, the 78 page long table of structural formulae and the 225 examples, there is no disclosure that A may be the 5-thiazolo[4,5-c]pyridinio moiety. United Kingdom Patent Specification No. 1,604,971 is concordant thereto and has a substantially identical disclosure. Published United Kingdom Patent Application No. 2,028,305A, although apparently not formally related, contains the same broad generic disclosure but exemplifies A only as hydrogen.

U.S. Patent No. 4,278,671 discloses 7-[2-(2-aminothiazol-4-yl)-2-(*syn*)-methoxyiminoacetamido]-cephalosporin derivatives of the formula

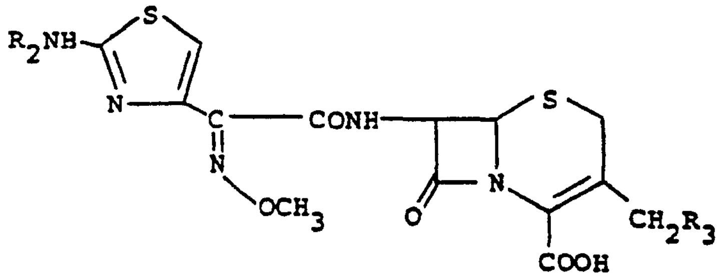

in which $R_2NH$ is an optionally protected amino group and $R_3$ is hydrogen or "the residue of a nucleophilic compound". The term "the residue of a nucleophilic compound" is broadly defined and it is then stated that $R^3$ "may alternatively be a quaternary ammonium group". Only pyridinium, variously substituted pyridinium, quinolinium, picolinium and lutidinium are disclosed as possible quaternary ammonium groups. There is no suggestion that the quaternary ammonium group may be the 5-thiazolo[4,5-c]pyridinio moiety. United Kingdom Patent Specification No. 1,581,854 is concordant thereto and has a substantially identical disclosure. Other patents to the same patentee, which are not formally related but which have similar disclosures, include U.S. Patent 4,098,888 and its divisionals U.S. Patents 4,203,899, 4,205,180, 4,298,606 and United Kingdom Patent Specification No. 1,536,281.

This invention relates to novel cephalosporin derivatives of the formula

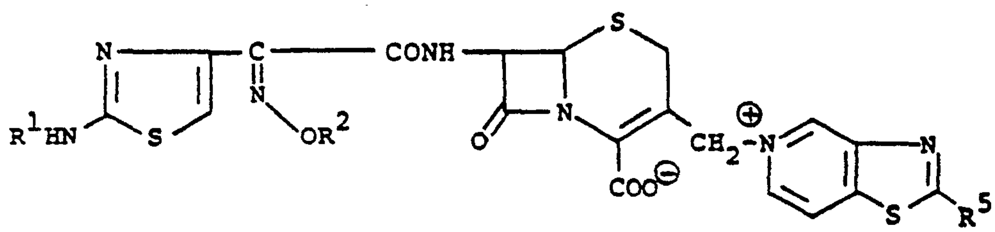

I

wherein $R^1$ is hydrogen or a conventional amino-protecting group, $R^2$ is a straight or branched chain alkyl group containing from 1 to 4 carbon atoms, allyl, 2-butenyl, 3-butenyl or a group of the formula

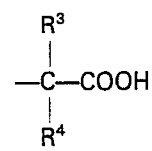

in which $R^3$ and $R^4$ each are independently hydrogen, methyl or ethyl, or $R^3$ and $R^4$, taken together with the carbon atom to which they are attached, may be a cycloalkylidene ring containing from 3 to 5 carbon atoms, and $R^5$ is hydrogen, amino, $C_1$—$C_6$-alkyl, $C_1$—$C_6$-alkoxy or $C_1$—$C_6$-alkylthio, and nontoxic pharmaceutically acceptable salts and physiologically hydrolyzable esters thereof, as well as processes for their preparation. Also included within the scope of this invention are the solvates (including hydrates) of the compounds of Formula I, as well as the tautomeric forms of the compounds of Formula I, e.g. the 2-iminothiazolin-4-yl form of the 2-aminothiazol-4-yl moiety.

As shown in the structural formula, the compounds of Formula I have the "syn" or "Z" configuration with respect to the alkoxyimino group. Because the compounds are geometric isomers, some of the "anti" isomer may also be present. This invention comprises compounds of Formula I containing at least 90% of the "syn" isomer. Preferably the compounds of Formula I are "syn" isomers which are essentially free of the corresponding "anti" isomers.

The nontoxic pharmaceutically acceptable acid addition salts of the compounds of Formula I include, for example, the salts with hydrochloric, hydrobromic, formic, nitric, sulfuric, methanesulfonic, phosphoric, acetic and trifluoroacetic acids, and other acids which have been used in the penicillin and cephalosporin art.

The compounds of Formula I in which $R^1$ is hydrogen exhibit high antibacterial activity against various Gram positive and Gram negative bacteria, and are useful in the treatment of bacterial infections in animals, including man. The compounds of Formula I may be formulated for parenteral use in a conventional manner utilizing known pharmaceutical carriers and excipients, and may be presented in unit dosage form or in multi-dosage containers. The compositions may be in the form of solutions, suspensions or emulsions in oily or aqueous vehicles, and may contain conventional dispersing, suspending or

stabilizing agents. The compositions may also be in the form of a dry powder for reconstitution before use, e.g. with sterile, pyrogen-free water. The compounds of Formula I may also be formulated as suppositories utilizing conventional suppository bases such as cocoa butter or other glycerides. The compounds of this invention may, if desired, be administered in combination with other antibiotics such as penicillins or other cephalosporins.

When provided in unit dosage forms the compositions will preferably contain from about 50 to about 1500 mg of the active ingredient of Formula I. The dosage of the compounds of Formula I is dependent on such factors as the weight and age of the patient as well as the particular nature and severity of the disease, and is within the discretion of the physician. However, the dosage for adult human treatment will usually be in the range of from about 500 to about 5000 mg per day, depending on the frequency and route of administration. When administered intramuscularly or intravenously to an adult human, a total dosage of from about 750 to about 3000 mg per day, in divided doses, normally will be sufficient, although higher daily doses of some of the compounds may be desirable in the case of *Pseudomonas* infections.

The preferred compounds of Formula I are those in which $R^1$ is hydrogen, $R^2$ is methyl, ethyl or a group of the formula

$$\begin{array}{c} R^3 \\ | \\ -C-COOH \\ | \\ R^4 \end{array}$$

in which $R^3$ and $R^4$ each are methyl, or $R^3$ and $R^4$, taken together with the carbon atom to which they are attached, are cyclopropylidene or cyclobutylidene, and $R^5$ is hydrogen or amino. Particularly preferred compounds are those in which $R^1$ is hydrogen,, $R^2$ is methyl, ethyl, 2-carboxyprop-2-oxyimino or 1-carboxycyclobut-1-oxyimino, and $R^5$ is hydrogen, methyl or amino. The most preferred compounds of the invention are

1) 7-[(Z)-2-(2-aminothiazol-4-yl)-2-methoxyiminoacetamido]-3-{(5-thiazolo[4,5-c]pyridinio)methyl}-3-cephem-4-carboxylate,

2) 7-[(Z)-2-(2-aminothiazol-4-yl)-2-(2-carboxyprop-2-oximino)-acetamido]-3-{(5-thiazolo[4,5-c]pyridinio)methyl}-3-cephem-4-carboxylate,

3) 7-[(Z)-2-(2-aminothiazol-4-yl)-2-ethoxyiminoacetamido]-3-{(5-thiazolo[4,5-c]pyridinio)methyl}-3-cephem-4-carboxylate,

4) 7-[(Z)-2-(2-aminothiazol-4-yl)-2-(1-carboxycyclobut-1-oxyimino)-acetamido]-3-{(5-thiazolo[4,5-c]pyridinio)methyl}-3-cephem-4-carboxylate,

5) 7-[(Z)-2-(2-aminothiazol-4-yl)-2-methoxyiminoacetamido]-3-[{(5-aminothiazolo[4,5-c]pyridinio)methyl)}methyl]-3-cephem-4-carboxylate, and

6) 7-[(Z)-2-(2-aminothiazol-4-yl)-2-methoxyiminoacetamido]-3-[{5-(2-methylthiazolo[4,5-c]pyridinio)}methyl-3-cephem-4-carboxylate.

In the primary evaluation of compounds of this invention, the Minimum Inhibitory Concentrations (MIC's) of the compounds were determined by the two-fold serial agar dilution method in Mueller-Hinton agar against 32 strains of test organisms in six groups. The geometric means of the MIC's determined in these tests are shown in Table 1.

TABLE 1

| Compound of Example | Geometric Mean of MIC (mcg)mL) | | | | | |
|---|---|---|---|---|---|---|
| | (G+)—Ia (5 strains) | (G+)—Ib (5) | (G−)—Ia (5) | (G−)—Ib (6) | (G−)—II (5) | (G−)—III (6) |
| 1 = (Ia) | 0.53 | 1.2 | 0.025 | 0.20 | 0.40 | 3.2 |
| 3 = (Ib) | 0.53 | 1.2 | 0.044 | 0.28 | 0.69 | 3.2 |
| 4 = (Ic) | 0.46 | 0.80 | 0.076 | 0.63 | 1.6 | 5.6 |
| 5 = (Id) | 5.5 | 11 | 0.050 | 0.89 | 2.4 | 1.8 |
| 7 = (Ie) | 3.1 | 6.1 | 0.033 | 0.71 | 1.4 | 1.8 |
| 8 = (If) | 0.53 | 1.2 | 0.017 | 0.16 | 0.6 | 3.2 |
| 9 = (Ig) | 2.7 | 6.3 | 0.038 | 0.79 | 2.4 | 2.5 |
| 10 = (Ih) | 0.53 | 1.2 | 0.011 | 0.089 | 0.46 | 3.5 |
| 11 = (Ii) | 0.46 | 0.4 | 0.05 | 0.25 | 1.0 | 11 |
| 12 = (Ij) | 1.2 | 1.6 | 0.066 | 0.56 | 1.4 | 10 |

(G+)—Ia: Penicillin-sensitive *S. aureus* (5 strains)
(G+)—Ib: Penicillin-resistant *S. aureus* (5 strains)
(G−)—Ia: Cephalothin-sensitive *E. coli* (2 strains), *Kl. pneumoniae* (1 strain) and *Pr. mirabilis* (2 strains)
(G−)—Ib: Cephalothin-resistant *E. coli* (3 strains) and *Kl. pneumoniae* (3 strains)
(G−)—II: *M. morganii* (1 strain), *Ent. cloacae* (2 strains) and *Ser. marcescens* (2 strains)
(G−)—III: *Ps. aeruginosa* (6 strains)

The absorption of some of the preferred compounds were determined in mice following a single intramuscular injection of the test compound (dissolved in 0.1M phosphate buffer; pH 7) at a dosage of 20 mg/kg. Blood samples were collected from the orbital sinuses into heparinized capillary tubes and assayed in Nutrient Agar (pH 6.6) using *E. coli* Ess-22-31 as the test organism. The blood levels at various time intervals, the half-life values ($t_{1/2}$) and the areas under the curve (AUC) are shown in Table 2.

TABLE 2

Mouse Blood Levels

| Compound | Mouse Blood Level (mcg/mL) Minutes After Administration 10 | 20 | 30 | 40 | 50 | 60 | 90 | 120 | T ½ (min) | AUC (mcg·hr/ml) |
|---|---|---|---|---|---|---|---|---|---|---|
| Ia | 20 | 15 | 11 | 8.6 | 6.9 | 6.5 | 3.1 | 2.1 | 35 | 15 |
| Ib | 15 | 12 | 8.4 | 6.1 | 3.8 | 0.92 | 0.68 | 0.43 | 19 | 8.4 |
| Ic | 18 | 14 | 10 | 8.2 | 5.8 | 3.6 | 1.9 | 1.3 | 28 | 12 |
| Id | 16 | 14 | 11 | 6.1 | 3.9 | 2.5 | 0.72 | <0.2 | 16 | 9.5 |
| Ie | 13 | 12 | 7.5 | 4.3 | 3.0 | 2.6 | 0.51 | 0.15 | 16 | 7.9 |
| If | 14 | 14 | 7.6 | 7.0 | 4.4 | 3.9 | 2.9 | 1.0 | 29 | 11 |
| Ig | 20 | 15 | 8.2 | 7.2 | 2.6 | 1.9 | 0.78 | 0.24 | 17 | 9.9 |
| Ih | 16 | 10 | 6.9 | 5.9 | 4.1 | 3.0 | 0.79 | 0.13 | 16 | 8.6 |
| Ii | 14 | 10 | 5.9 | 4.8 | 2.4 | 1.5 | 0.25 | <0.1 | 13 | 6.8 |

The *in vitro* activity of some of the preferred compounds against 30 strains of fastidious bacteria was determined by the two-fold serial agar dilution method in Gonococcus agar (GC agar), and the results are shown in Table 3.

TABLE 3

*In Vitro* Activity of Preferred Compounds Against Fastidious Bacteria

| Test Organism | Geometric Mean of MIC (mcg/mL) | | |
|---|---|---|---|
| | Ia | Id | Ie |
| *S. pyogenes* (5 strains) | 0.0125 | 0.20 | 0.20 |
| *S. pneumoniae* (5) | 0.0125 | 0.20 | 0.20 |
| *N. gonorrhoeae* (5) | 0.0125 | 0.10 | 0.10 |
| *N. meningitidis* (5) | 0.0125 | 0.10 | 0.10 |
| *H. influenzae* (7) (ampicillin sensitive) | 0.0125 | 0.10 | 0.10 |
| *H. influenzae* (3) (ampicillin resistant) | 0.0125 | 0.10 | 0.10 |

The median protective dose ($PD_{50}$) of some of the preferred compounds against various microorganisms was determined in mice by intramuscular administration of the test compound immediately after an intraperitoneal bacterial challenge. The results are shown in Table 4.

TABLE 4

*In vivo* Activity

| Microorganism | $PD_{50}$ (mg/kg, im) | | | | |
|---|---|---|---|---|---|
| | Compound | | | | |
| | Ia | Ic | Id | Ie | If |
| *S. aureus* Smith | 0.45 | 1.1 | 7.2 | 3.1 | 0.62 |
| *S. aureus* BX—1633 | 0.75 | — | 21 | — | — |
| *E. coli* Juhl | 0.012 | 0.048 | 0.082 | 0.08 | 0.015 |
| *P. aeruginosa* A9843 | 7.2 | 8.6 | 4.3 | 1.8 | 2 |
| *P. aeruginosa* A21509 | 6.25 | — | 5.0 | — | — |
| *S. pyogenes* A20201 | — | — | 0.23 | — | — |

In another aspect, this invention relates to processes for the preparation of the compounds of Formula I. There are two basic procedures for converting a readily available starting cephalosporin to another cephalosporin having different substituents on the 7- and 3-positions. One may first remove the 7-substituent and replace it with the desired 7-substituent, and then insert the desired 3-substituent. Alternatively, one may first insert the desired 3-substituent and subsequently exchange the 7-substituent. The compounds of Formula I may be prepared by either procedure and both are included within the scope of this invention, but it is preferred to insert the desired 7-substituent first and then insert the desired 3-substituent. The preferred procedure is shown below in Reaction Scheme 1 while the alternative procedure

is shown in Reaction Scheme 2. In each reaction scheme, n may be 0 or 1. The abbreviation "Tr" represents the trityl (triphenylmethyl) group, which is a preferred amino-protecting group. The abbreviation "Ph" represents the phenyl group. Thus, the —$CH(Ph)_2$ moiety is the benzhydryl group, which is a preferred carboxyl-protecting group.

## Reaction Scheme 1

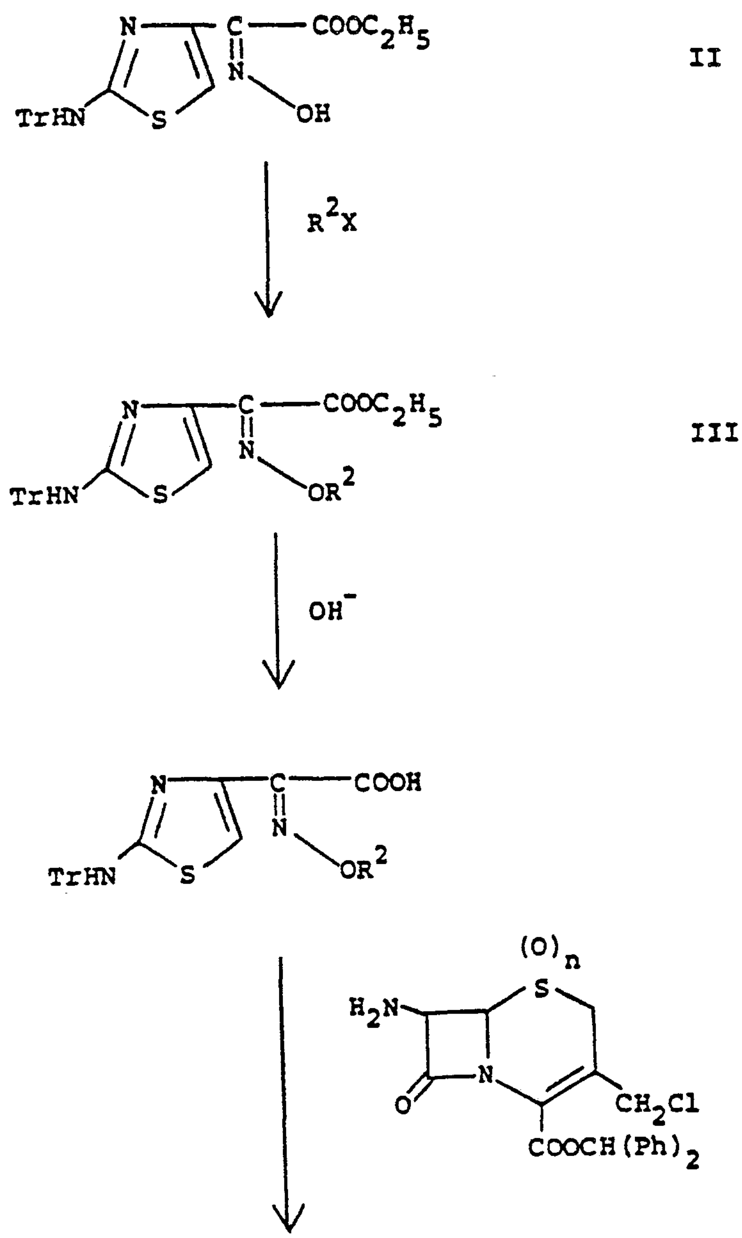


II

III

IV

V

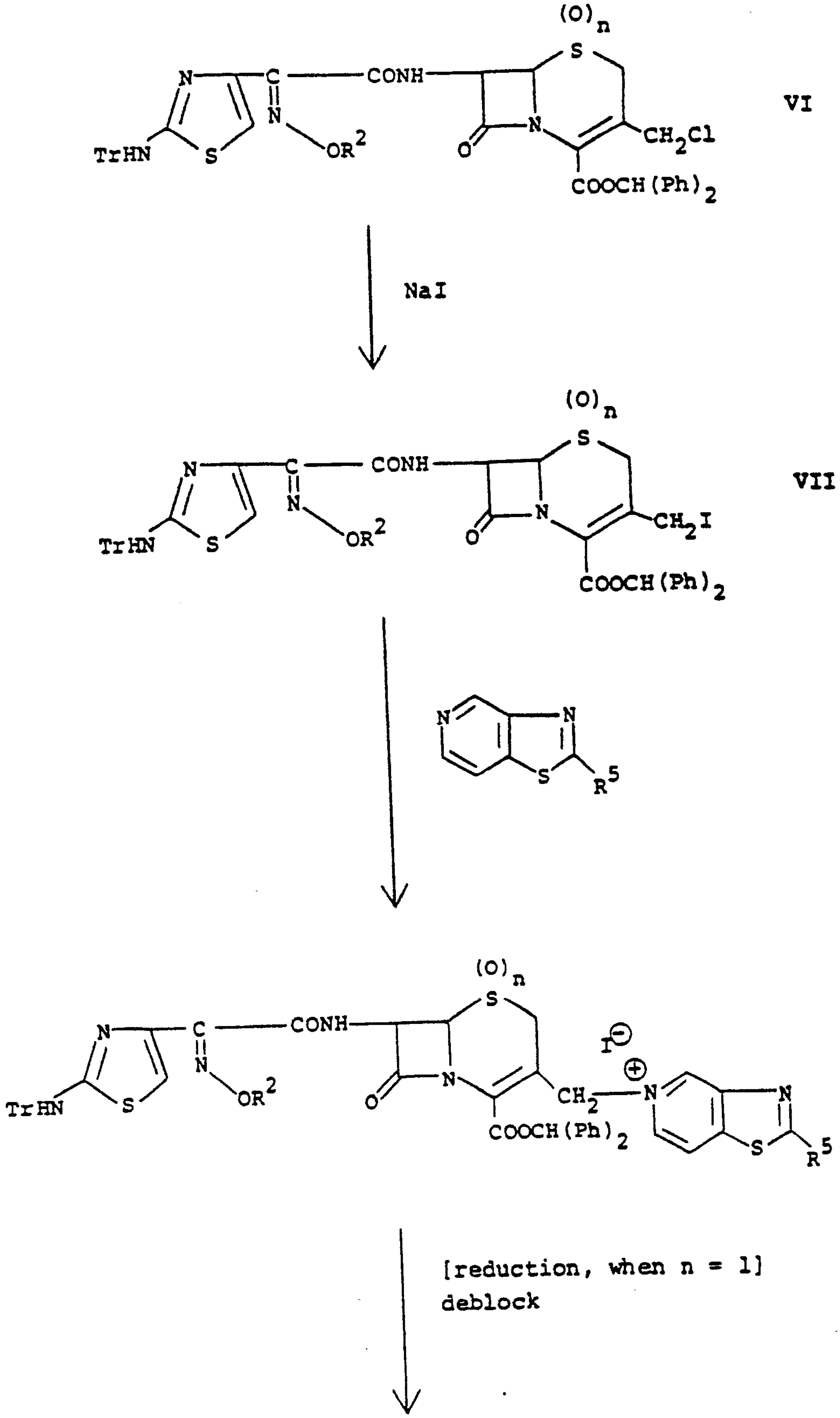


VI

VII

XIII

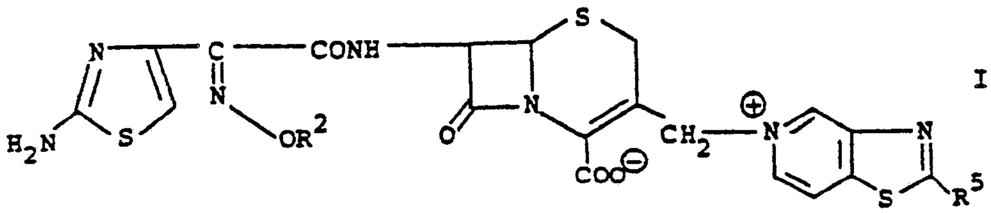

I

<u>Reaction Scheme 2</u>

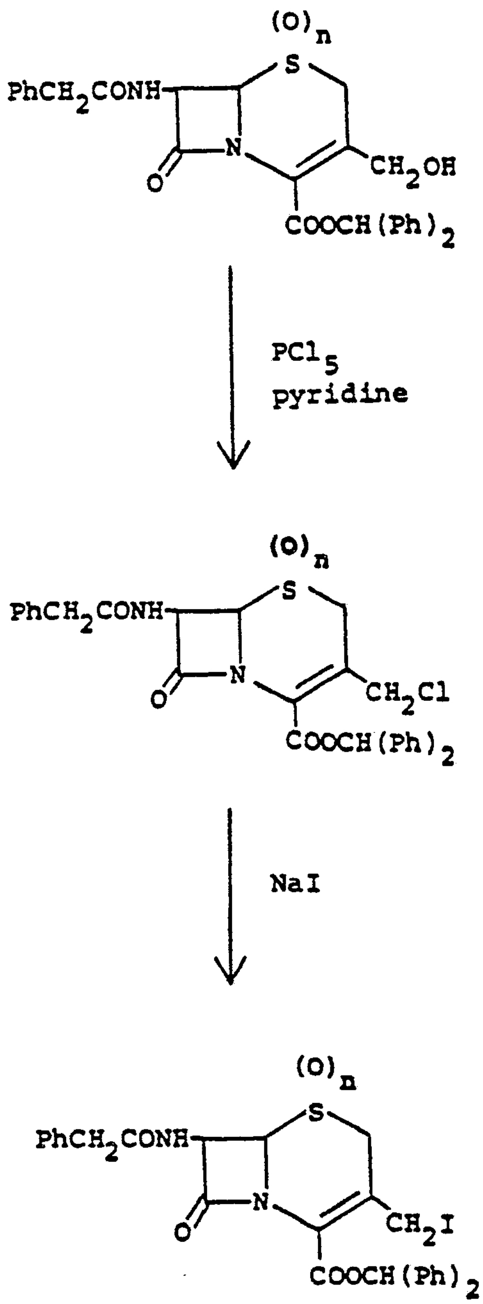


VIII

IX

X

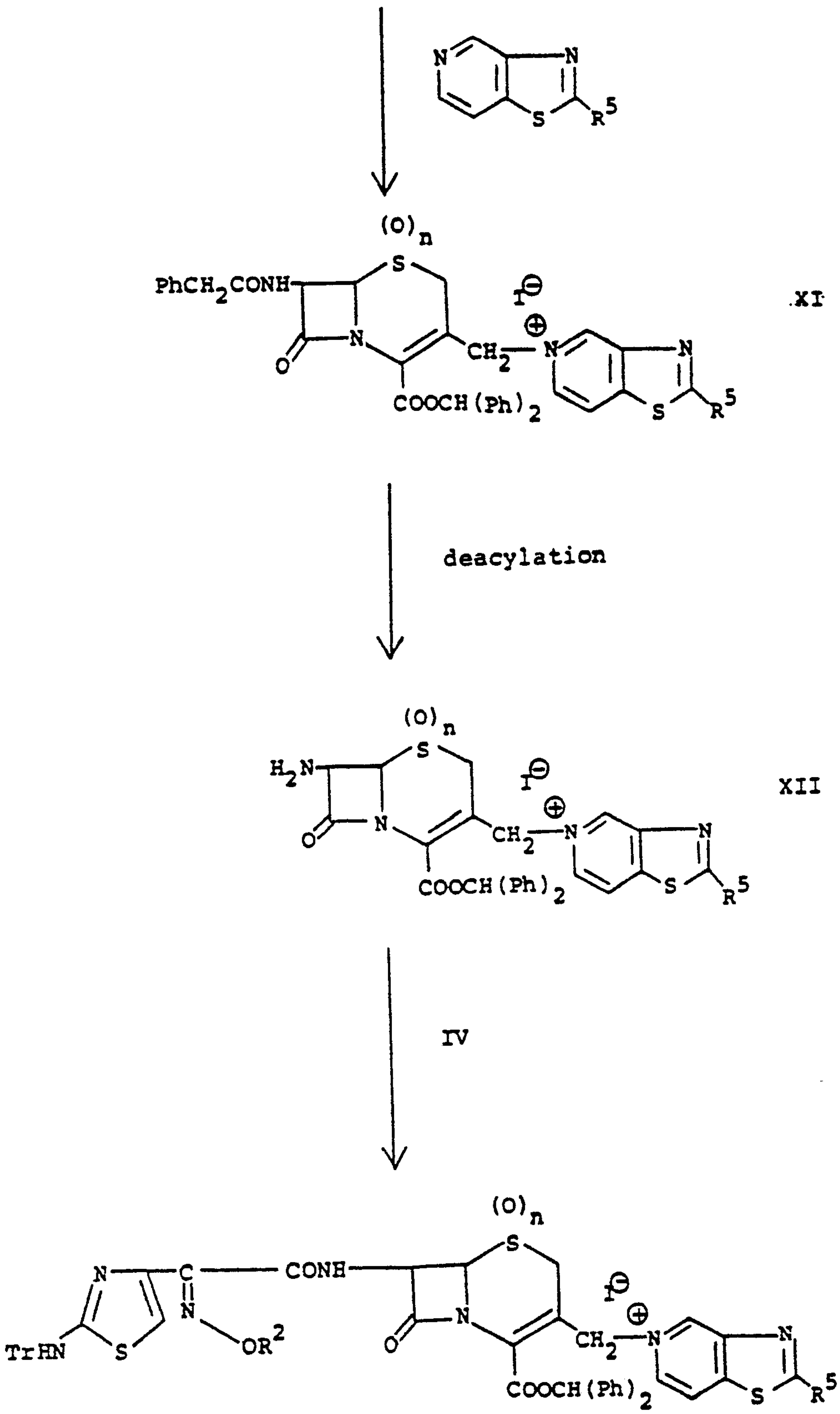


XI

XII

XIII

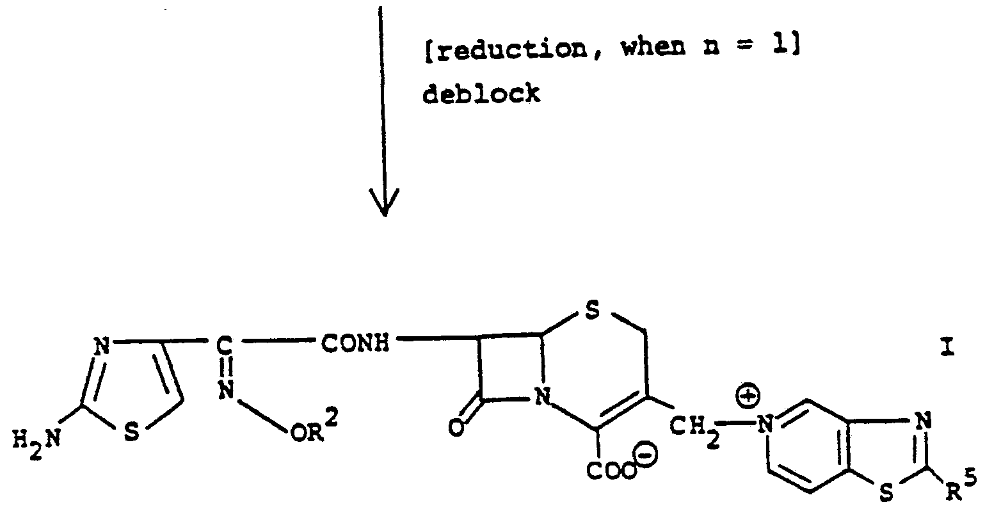


I

Although the above Reaction Schemes show preferred multi-step procedures for the preparation of the compounds of Formula I, it will be appreciated that other starting materials and procedures may be utilized to prepare the intermediates used in the key step of each Reaction Scheme. Thus, the key step in Reaction Scheme 1 is the reaction of Compound VII with the thiazolo[4,5-c]pyridine. Compound VII may itself be prepared by other procedures. Similarly, the key step in Reaction Scheme 2 is the acylation of Compound XII with Compound IV. Both compounds XII and IV may be prepared by other procedures.

The present invention provides a process for the preparation of compounds of the formula

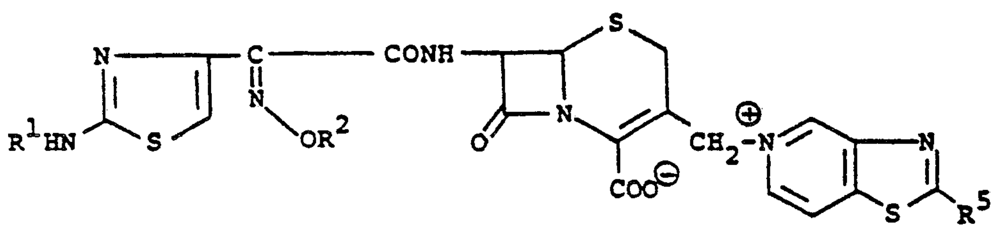

I

wherein $R^1$, $R^2$ and $R^5$ are as defined above and nontoxic pharmaceutically acceptable salts, physiologically hydrolyzable esters and solvates thereof, which process comprises reacting a compound of the formula

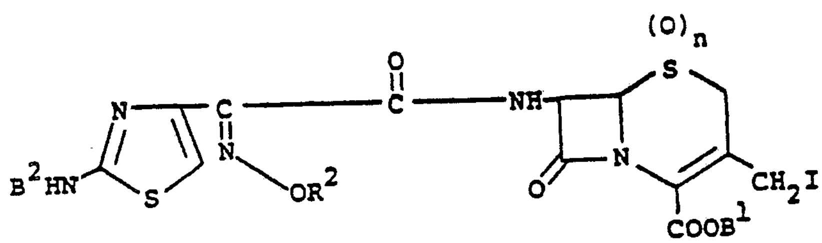

XIV

in which $R^2$ is as defined above, $B^1$ is a conventional carboxyl-protecting group, $B^2$ is a conventional amino-protecting group and n is 0 or 1, with a thiazolo[4,5-c]pyridine of the formula

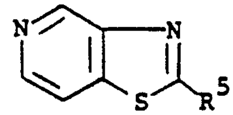

III

in which $R^5$ is as defined above, to produce a compound of the formula

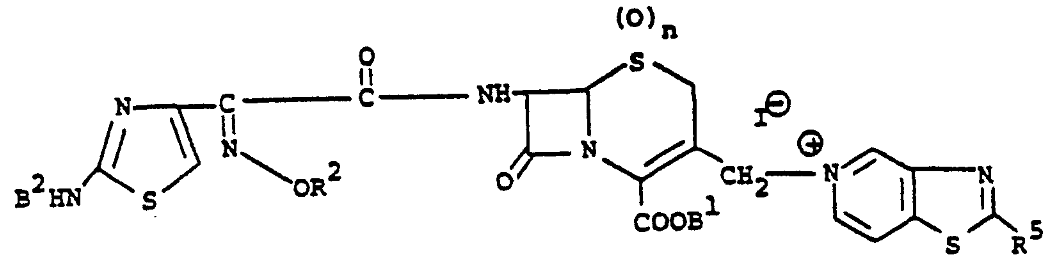

XV

and, if n is 1, reducing the sulfoxide by conventional means, and subsequently removing all protecting groups by conventional means.

The reaction is carried out in a non-aqueous organic solvent such as dimethyl sulfoxide, hexamethylphosphoramide, methylene chloride, chloroform, ethyl ether, hexane, ethyl acetate, tetrahydrofuran, acetonitrile and the like, or mixtures of such solvents. The reaction is conveniently carried out at a temperature of from about −10°C to about +50°C; we normally prefer to conduct the reaction at room temperature. At least one mole of the tertiary amine should be used per mole of Compound XIV; we normally prefer to utilize from about 50% to 100% excess of the thiazolo[4,5-c]pyridine.

Carboxyl-protecting groups suitable for use as $B^1$ in the above reaction are well-known to those skilled in the art and include aralkyl groups such as benzyl, p-methoxybenzyl, p-nitrobenzyl and diphenylmethyl (benzhydryl); alkyl groups such as t-butyl; haloalkyl groups such as 2,2,2-trichloroethyl, and other carboxyl-protecting groups described in the literature, e.g. in U.K. Patent 1,399,086. We prefer to utilize carboxyl-protecting groups which are readily removed by treatment with acid. Particularly preferred carboxyl-protecting groups are the benzhydryl and t-butyl moieties.

Amino-protecting groups suitable for use as $B^2$ are also well-known in the art, and include the trityl group and acyl groups such as chloroacetyl, formyl and trichloroethoxycarbonyl. Amino-protecting groups which are readily removed by treatment with acid, e.g. the trityl group, are preferred.

The present invention also provides a process for the preparation of compounds of the formula

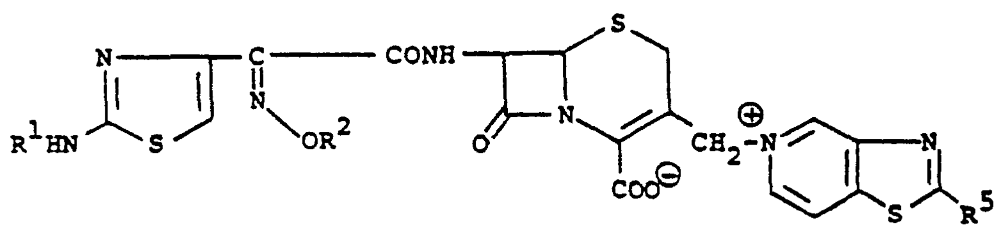

I

wherein $R^1$, $R^2$ and $R^5$ are as defined above and nontoxic pharmaceutically acceptable salts, physiologically hydrolyzable esters and solvates thereof, which process comprises acylating a compound of the formula

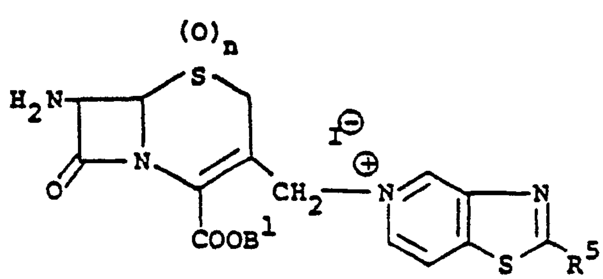

XVI

or an N-silyl derivative thereof, in which $B^1$ is hydrogen or a conventional carboxyl-protecting group, n is 0 or 1 and $R^5$ is as defined above, with an acylating derivative of an acid of the formula

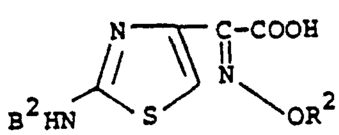

XVII·

wherein $B^2$ is a conventional amino-protecting group and $R^2$ is as defined above, to produce a compound of the formula

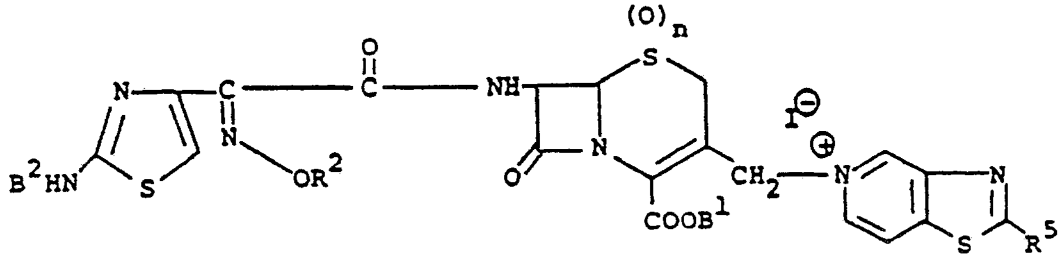

XV

and, if n is 1, reducing the sulfoxide by conventional means, and subsequently removing all protecting groups.

The acylating derivatives of the acid of Formula XVII include the acid halides (and particularly the acid chloride), mixed acid anhydrides (such as the acid anhydrides formed with pivalic acid or a haloformate such as ethyl chloroformate), and activated esters (such as may be formed with N-hydroxybenztriazole in the presence of a condensing agent such as dicyclohexylcarbodiimide). The acylation may also be effected by use of the free acid of Formula XVII in the presence of a condensing agent such as dicyclohexylcarbodiimide, carbonyldiimidazole or an isoxazolium salt. As used herein, the term "acylating derivative" of the acid of Formula XVII includes the free acid itself in the presence of a condensing agent such as described above. The preferred acylating derivative of the acid of Formula XVII is the acid chloride, preferably used in the presence of an acid binding agent (and particularly a tertiary amine acid binding agent such as triethylamine, dimethylaniline or pyridine).

When the acylation is conducted with an acid halide it is possible to utilize an aqueous reaction medium, but a non-aqueous medium is preferred. When acid anhydrides, activated esters, or the free acid in the presence of a condensing agent, are used for the acylation, the reaction medium should be non-aqueous. Particularly preferred solvents for the acylation reaction are halogenated hydrocarbons such as methylene chloride and chloroform, but tertiary amides such as dimethylacetamide or dimethylformamide may be utilized, as well as other conventional solvents such as tetrahydrofuran, acetonitrile and the like.

The acylation reaction may be conducted at a temperature of from about −50°C to about +50°C. However, it is preferably conducted at or below room temperature and most preferably from about −30°C to about 0°C. It is usually preferred to acylate the compound of Formula XVI with about a stoichiometric amount of the acylating agent of Formula XVII, although a small excess (e.g. 5—25%) of the acylating agent may be utilized.

It is preferable that the compound of Formula XVI be acylated in the form of its N-silyl derivative (when utilizing a non-aqueous reaction medium). This is conveniently done *in situ* by simply adding a suitable silylating agent (e.g. N,O-bistrimethylsilylacetamide) to the solution of Compound XVI prior to the addition of the acylating agent of Formula XVII. We prefer to utilize about 3 moles of silylating agent per mole of Compound XVI although this is not critical. The silyl compound is readily removed after acylation by the addition of water.

When substituent $R^2$ of the acylating acid of Formula XVII is a group of the formula

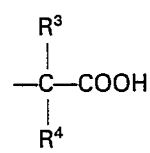

it is preferred that the acylating acid contain a carboxyl-protecting group, i.e. that the acylating acid be utilized in the form

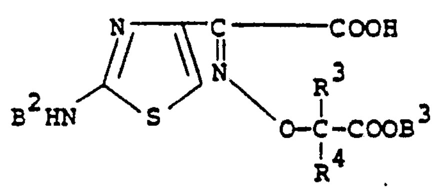

XVIII

in which $B^2$ is a conventional amino-protecting group as described above, and $B^3$ is a conventional carboxyl-protecting group as described above for $B^1$.

The acylating acids of Formula XVII (and the corresponding precursor esters), including the carboxyl- and amino-protected derivatives [XVIII] thereof, are known in the art or may be prepared by known procedures. Thus, Compounds IIIa, IIIb and IIIc (shown in Preparation No. 1, below) and Compounds IVa, IVb and IVc (shown in Preparation No. 2, below) are described in Tetrahedron, *34*, 2233—2243 (1978), in which they were prepared by a different route. Compounds IVd' and IVe', used as starting materials in

Preparation No. 11 and Preparation No. 13, respectively, were prepared according to the general procedure described in U.S. Patent No. 4,258,041 and published United Kingdom Patent Application No. 2,025,398.

The thiazolo[4,5-c]pyridines of the formula

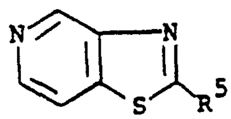

wherein $R^5$ is as described above are known compounds. The preparation of the compounds in which $R^5$ is hydrogen or methyl is described in Pharm. Bull. (Japan), *2*, 196—200 (1954) [Chemical Abstracts, *50*, 1000i]. The preparation of the compound in which $R^5$ is amino is described in Pharm. Bull. (Japan), *2*, 34—37 (1954) [Chemical Abstracts, *50*, 335h]. The compound in which $R^5$ is methylthio is described in J. Heterocyclic Chem., *14*, 1045 (1977).

When utilizing Reaction Scheme 1 or 2 in which the cephalosporin nucleus is in the form of the 1-oxide (n = 1), the 1-oxide is prepared by known procedures such as oxidation with m-chloroperbenzoic acid, peracetic acid, etc. The 1-oxide subsequently may be reduced by known procedures, e.g. reduction of the corresponding alkoxysulfonium salt with iodide ion in an aqueous medium. The alkoxysulfonium salt itself is readily prepared by treatment of the 1-oxide with, for example, acetyl chloride.

As used herein, the terms "(lower)alkyl" and "(lower)alkoxy" mean straight or branched chain alkyl and alkoxy groups containing from 1 to 6 carbon atoms, inclusive.

### Preparation No. 1

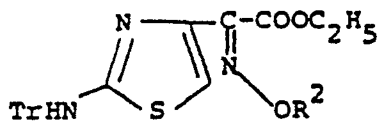

III

Ethyl (Z)-2-Methoxyimino-2-(2-tritylaminothiazol-4-yl)acetate (IIIa)

A mixture of ethyl (Z)-2-hydroxyimino-2-(2-tritylaminothiazol-4-yl) acetate (II) (5.00 g, 10.9 mmoles), $CH_3I$ (2.04 mL, 32.8 mmoles) and $K_2CO_3$ (4.54 g, 32.8 mmoles) in dry dimethylsulfoxide (DMSO) (100 mL) was stirred at room temperature overnight and then poured into water (250 mL). The precipitate which formed was collected by filtration, washed with water and dried to give the title compound (5.15 g, quantitative yield). Mp. 115°C (dec.).

NMR: $\delta^{CTCl_3}$ ppm 1.32 (3H, t), 3.98 (3H, s), 4.30 (2H, q), 6.42 (1H, s), 7.2 (1H, m), 7.25 (15H, s).

Compounds IIIb, IIIc, IIId' and IIIe' were prepared by the general procedure set forth above, but replacing the methyl iodide with the appropriate iodide.

| Compound | $R^2$ | Yield (%) | Mp (°C) | Literature Mp. (°C) |
|---|---|---|---|---|
| IIIa | methyl | 100 | 115° (dec.) | ca. 120° (dec.)(1) |
| IIIb | ethyl | 67 | 97—98° | * |
| IIIc | allyl | * | * | * |
| IIId' | $—C(CH_3)_2COOtButyl$ | 100 | 125—126° | 123.5—125(2); 134(3) |
| IIIe' | 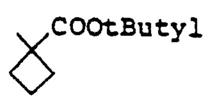 | 68 | 81—83° | not reported(3) |

*The ester was hydrolyzed without isolation
(1)Tetrahydron, *34*, 2233 (1978)
(2)U.S. Patent 4,258,041
(3)U.S. Patent 4,288,434

Preparation No. 2

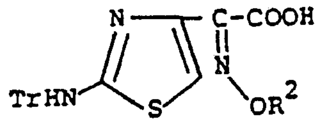

IV

(Z)-2-Methoxyimino-2-(2-tritylaminothiazol-4-yl)acetic acid (IVa)

The ethyl ester IIIa prepared in Preparation No. 1 (6.00 g, 12.7 mmoles) in ethanol (120 mL) was treated with 2N NaOH (12.7 mL) at room temperature overnight. The reaction mixture was adjusted to pH 8 by the addition of powdered dry ice and the solvent was evaporated under reduced pressure. The residue was dissolved in water (100 mL) and the solution was acidified with 1N HCl to pH 2 and then extracted with ethyl acetate (3 × 50 mL). The combined extracts were washed with a saturated aqueous NaCl solution, dried and evaporated. The residue was crystallized from ethyl acetate-hexane to afford 5.56 g (yield 98%) of the title product. Mp. 138—143°C (dec.).

NMR: $\delta^{CTCl_3}$ ppm 3.89 (3H, s), 6.52 (1H, s), 7.2 (15H, s).

Compounds IVb, IVc, IVd′ and IVe′ were prepared by the general procedure set forth above.

| Compound | $R^2$ | Yield (%) | Mp (°C, dec.) | Literature Mp (°C, dec.) |
|---|---|---|---|---|
| IVa | methyl | 98 | 138—143 | ca. 140[(1)] |
| IVb | ethyl | 85 | 140—145 | not reported[(1)] |
| IVc | allyl | 66 | 170—178 | ca. 170[(1)] |
| IVd′ | $—C(CH_3)_2COOtButyl$ | 77 | 174—175 | 152—156[(2)]; 190[(3)] |
| IVe′ | 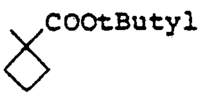 | 78 | 163—164 | not reported[(2)] |

[(1)]Tetrahedron, *34*, 2233 (1978)
[(2)]U.S. Patent 4,258,041
[(3)]U.S. Patent 4,288,434

Preparation No. 3

Benzhydryl 3-Hydroxymethyl-7-phenylacetamido-3-cephem-4-carboxylate (VIII)

To a stirred suspension of phosphate buffer (pH 7, 162.5 mL) and wheat bran (20 g, dry) at room temperature was added 7-phenylacetamidocephalosporanic acid sodium salt (5 gm, 12.1 mmoles) in one portion. The progress of the reaction was monitored by HPLC until the hydrolysis was complete (5 hours). The suspension was filtered to remove the wheat bran and the filtrate was cooled to 5—10°C for extractive esterification. To the cooled solution was added methylene chloride (32 mL) followed by a 0.5M solution of diphenyldiazomethane in methylene chloride (24 mL). The pH was then adjusted to 3.0 with 28% phosphoric acid. After 1 hour the reaction mixture was allowed to rise to 20°C. Heptane (56 mL) was slowly added and the resulting crystalline title product was recovered by filtration. Yield of the title product was 3.0 gm (50%).

Preparation No. 4

Benzhydryl 7-Amino-3-chloromethyl-3-cephem-4-carboxylate (V)

To a slurry of $PCl_5$ (8.3 g, 40 mmoles) in $CH_2Cl_2$ (100 mL) was added pyridine (3.2 g, 40 mmoles) and the mixture was stirred for 20 minutes at 20°C. To the mixture was added benzhydryl 3-hydroxymethyl-7-phenylacetamido-3-cephem-4-carboxylate prepared in Preparation No. 3 (5.1 g, 10 mmoles) with stirring at −40°C, in one portion. The mixture was stirred at −10°C for 15 minutes and allowed to stand at −10°C to −15°C for 7 hours. To the cooled solution (−20°C) was added propane-1,3-diol (10 mL) and the mixture was allowed to stand at −20°C for 16 hours and then at room temperature for 20 minutes with stirring. The resulting solution was washed with ice-water (2 × 20 mL) and saturated aqueous NaCl (10 mL), dried over $MgSO_4$ and concentrated *in vacuo*. The gummy residue (12 g) was dissolved in a mixture of $CHCl_3$ and n-hexane (2:1), and subjected to chromatography using a silica gel column (200 g) and the same solvent as eluant. Fractions containing the title compound were evaporated *in vacuo* and the residue triturated with n-hexane to give the title product (2.1 g, 51%), melting at >110°C (dec.).

IR: $\nu_{KBr}$ 3400, 2800, 1785, 1725 $cm^{-1}$.

UV: $\lambda_{max}^{EtOH}$ 265 nm ($E_{1cm}^{1\%}$ 160).

NMR: $\delta_{ppm}^{DMSO-d_6+CDCl_3}$ 3.69 (2H, s), 4.43 (2H, s), 5.09 (1H, d, J=4.5Hz), 5.24 (1H, d, J=4.5Hz), 6.87 (1H, s), 7.3 (10H, m).

Preparation No. 5

Benzhydryl 3-Chloromethyl-7-[(Z)-2-methoxyimino-2-(2-tritylaminothiazol-4-yl)acetamido]-3-cephem-4-carboxylate (VIa)

Benzhydryl 7-amino-3-chloromethyl-3-cephem-4-carboxylate prepared in Preparation No. 4 (2.29 g, 5.52 mmoles) in $CH_3CN$ (57 mL) was treated with bis(trimethylsilyl)acetamide (BSA, 4.09 mL, 16.6 mmoles) at room temperature for 50 minutes to give a clear solution. To the solution was added an acid chloride solution, which was prepared from (Z)-2-methoxyimino-2-(2-tritylaminothiazol-4-yl)acetic acid (IVa) (2.04 g, 4.60 mmoles) and $PCl_5$ (1.15 g, 5.52 mmoles) in methylene chloride (20 mL). The mixture was stirred at room temperature for 30 minutes, poured into cold water (200 mL) and extracted with ethyl acetate (3 × 100 mL). The combined extracts were washed with aqueous NaCl, dried and evaporated. The residual syrup (4 g) was chromatographed on a silica gel (150 g) column by eluting with 10:1 and 3:1 mixtures of toluene and ethyl acetate successively. The fractions containing the desired compound were combined and evaporated to afford 2.61 g (68%) of VIa as an amorphous powder.

NMR: $\delta^{CDCl_3}$ ppm 3.50 (2H, s), 4.02 (3H, s), 4.33 (2H, s), 4.98 (1H, d), 5.87 (1H, q), 6.65 (1H, s), 6.90 (1H, s), 7.3 (25H, m).

Preparation No. 6

Benzhydryl 3-Iodomethyl-7-[(Z)-2-methoxyimino-2-(2-tritylaminothiazol-4-yl)acetamido]-3-cephem'-4-carboxylate (VIIa)

A mixture of the 3-chloromethyl derivative prepared in Preparation No. 5 (VIa) (1.50 g, 1.79 mmoles) and NaI (1.34 g, 8.93 mmoles) in methyl ethyl ketone (30 mL) was stirred at room temperature for 1 hour. After evaporation of the solvent the residue was dissolved in ethyl acetate (100 mL) and washed with water, aqueous $Na_2S_2O_3$ and aqueous NaCl, dried and evaporated to give the title compound VIIa (1.47 g, 89%) as an amorphous powder.

NMR: $\delta^{CDCl_3}$ ppm 3.55 (2H, ABq), 4.00 (3H, s), 4.25 (2H, s), 4.97 (1H, d, 5.80 (1H, q), 6.65 (1H, s), 6.90 (1H, s), 7.3 (25H, m).

Preparatiion No. 7

Benzhydryl 3-Chloromethyl-7-[(Z)-2-ethoxyimino-2-(2-tritylaminothiazol-4-yl)acetamido]-3-cephem-4-carboxylate (VIb)

To a solution of (Z)-2-ethoxyimino-2-(2-tritylaminothiazol-4-yl)acetic acid (IVb) (1.095 g, 2.4 mmoles) in dichloromethane (20 mL) was added phosphorus pentachloride (500 mg). After stirring for 1 hour at room temperature, the mixture was added in one portion to an ice-cooled solution of Compound V (1.083 g, 2.4 mmoles) and BSA (1 mL) in dichloromethane (20 mL). After stirring for 0.54 hour the reaction mixture was poured into 10% aqueous $NaHCO_3$ (200 mL) and extracted with $CHCl_3$ (100 mL). The extract was washed with water, dried over $MgSO_4$, and evaporated under reduced pressure. The residue was chromatographed on a silica gel column. Elution with $CHCl_3$ gave VIb as an amorphous powder, 1.76 g (86%).

NMR: $\delta^{CDCl_3}$ ppm 1.40 (3H, t, $CH_2CH_3$), 3.53 (2H, ABq, 2-$CH_2$), 4.37 (2H, s, —$CH_2Cl$), 4.60 (2H, q, —$CH_2CH_3$), 4.90 (1H, d, 6-H), 5.89 (1H, d, 7-H), 6.88 (1H, s, thiazole-H), 6.91 (1H, s, benzhydryl-CH).

Preparation No. 8

Diphenylmethyl 7-[(Z)-2-Ethoxyimino-2-(2-tritylaminothiazol-4-yl)acetamido]-3-iodomethyl-3-cephem-4-carboxylate (VIIb)

A mixture of VIb prepared in Preparation No. 7 (1.07 g, 1.25 mmoles) and NaI (562 mg, 2.75 mmoles) in acetone (20 mL) was stirred for 1 hour. The mixture was filtered and the filtrate was poured into water and extracted with ethyl acetate. The organic layer was washed successively with 5% aqueous $Na_2S_2O_3$, water and saturated aqueous NaCl, dried over $MgSO_4$ and evaporated to give 1.04 g (89%) of Compound VIIb.

NMR: $\delta^{CDCl_3}$ ppm 3.55 (2H, q, 2-$CH_2$), 4.27 (2H, s, $CH_2I$), 5.02 (1H, d, 6-H), 5.87 (1H, d, 7-H), 6.68 (1H, s, thiazole ring H), 6.93 (1H, s, benzylhydryl-CH).

Preparation No. 9

Benzhydryl 7-[(Z)-2-Allyloxyimino-2-(2-tritylaminothiazol-4-yl)acetamido]-3-chloromethyl-3-cephem-4-carboxylate (VIc)

To a suspension of Compound V (1.35 g, 3 mmoles) in methylene chloride (20 mL) was added BSA (1.1 mL, 4.5 mmoles), and the mixture was stirred for 30 minutes at room temperature to become a clear solution. A mixture of (Z)-2-allyloxyimino-2-(2-tritylaminothiazol-4-yl)acetic acid (IVc) (1.40 g, 3.0 mmoles) and phosphorus pentachloride (690 mg, 3.3 mmoles) in methylene chloride (20 mL) was stirred for 15 minutes at room temperature and poured in one portion into the solution of the trimethylsilylated Compound V. The mixture was stirred for 20 minutes at room temperature and diluted with ethyl acetate (200 mL), washed with aqueous sodium bicarbonate and water, dried and evaporated under reduced pressure. The oily residue was purified by silica gel column chromatography (Wako-gel, C-200, 30 g). The column was eluted with chloroform and the fractions containing the desired product were combined.

Evaporation under reduced pressure afforded the title compound (VIc) as an amorphous powder, yield 2.32 g (89%). Mp. 100—115°C (dec.).

IR: $\nu_{max}^{KBr}$ $cm^{-1}$ 3390, 1790, 1730, 1680, 1530, 1380, 1250, 1160, 1020.

NMR: $\delta^{CDCl_3}$ ppm 3.50 (2H, 2-H), 4.32 (2H, s, 3-$CH_2$), 4.6—6.1 (7H, m, $CH_2CH{=}CH_2$ and 6,7-H), 6.70 (1H, s, thiazole-H), 6.90 (1H, s, $Ph_2CH$), 7.1—7.6 (30H, m, phenyl protons).

Anal. Calc'd. for $C_{48}H_{40}N_5O_5S_2Cl \cdot 1/3CHCl_3$: C, 64.05; H, 4.45; N, 7.73; S, 7.08; Cl, 7.82.

Found: C, 64.13, 63.99; H, 4.61, 4.64; N, 7.50, 7.30; S, 6.85, 6.85; Cl, 7.55, 7.46.

Preparation No. 10

Benzhydryl 7-[(Z)-2-Allyloxyimino-2-(tritylaminothiazol)-4-yl)acetamido]-3-iodomethyl-3-cephem-4-carboxylate (VIIc)

A mixture of Compound VIc (2.30 g, 2.65 mmoles) and sodium iodide (2 g, 13.3 mmoles) in acetone (15 mL) was stirred for 1 hour at room temperature and then evaporated under reduced pressure. A solution of the oily residue in ethyl acetate (200 mL) was washed with 10% sodium thiosulfate and water, evaporated under reduced pressure to afford Compound VIIc as an amorphous powder, which was used in the subsequent step without further purification. Yield 2.52 g (99%).

Preparation No. 11

Benzhydryl 3-Chloromethyl-7-[(Z)-2-(2-t-butoxycarbonylprop-2-oxyimino)-2-(2-tritylaminothiazol-4-yl)acetamido]-3-cephem-4-carboxylate (VId')

*Procedure 1*

A mixture of (Z)-2-(2-t-butoxycarbonylprop-2-oxyimino)-2-(2-tritylaminothiazol-4-yl)acetic acid (IVd') (1.94 g, 3.6 mmoles) DCC (742 mg, 3.6 mmoles) and N-hydroxybenztriazole (486 mg, 3.6 mmoles) in tetrahydrofuran (THF) (45 mL) was stirred at room temperature for 45 minutes, during which dicyclohexylurea separated. The dicyclohexylurea was removed by filtration and the filtrate was mixed with V (1.5 g, 3.6 mmoles). The mixture was stirred overnight at room temperature and then evaporated *in vacuo*. The residual oil was dissolved in $CHCl_3$ (20 mL), washed with saturated aqueous $NaHCO_3$ and saturated aqueous NaCl, dried over $MgSO_4$ and evaporated to dryness. The residue (3.9 g) was dissolved in n-hexane:$CHCl_3$ (1:2) and passed through a silica gel column (40 g) using the same solvent system. Fractions containing the title compound were evaporated *in vacuo* to give 1.3 g (39%) of VId' melting at >100°C (dec.).

IR: $\nu_{max}^{KBr}$ $cm^{-1}$ 3390, 1790, 1715, 1690.

UV: $\lambda_{max}^{EtOH}$ 240 ($E_{1\,cm}^{1\%}$ 280), 265 ($E_{1\,cm}^{1\%}$ 190).

NMR: $\delta^{CDCl_3}$ ppm 1.45 (9H, s), 1.63 & 1.66 (6H, each s), 3.49 (2H, broad s), 4.34 (2H, s), 4.96 (1H, d, J=4.5Hz), 5.90 (1H, d-d, J=4.5 & 7.5), 6.66 (1H, s), 6.86 (1H, s), 7.0—7.5 (25H, m), 8.23 (1H, d, J=7.5Hz).

*Procedure 2*

A solution of V (1.86 g, 4.49 mmoles) in $CH_3CN$ (46.5 mL) was treated with BSA (3.33 mL, 13.5 mmoles) at room temperature for 50 minutes to give a clear solution. To the solution was added an acid chloride solution which had been prepared from IVd' (2.56 g, 4.49 mmoles) and $PCl_5$ (1.12 g, 5.38 mmoles) in methylene chloride (26 mL). The mixture was stirred at room temperature for 30 minutes, poured into cold water (100 mL) and extracted with ethyl acetate (3 × 50 mL). The combined extracts were washed with aqueous NaCl, dried and evaporated. The residual syrup (5 g) was chromatographed on a silica gel (100 g) column by eluting with 10:1 mixture of toluene and ethyl acetate. The fractions containing the desired compound were combined and evaporated to afford 2.84 g (65% of VId'.

Preparation No. 12

Benzhydryl 7-[(Z)-2-(2-t-Butoxycarbonylprop-2-oxyimino)-2-(2-tritylaminothiazol-4-yl)acetamido]-3-iodomethyl-3-cephem-4-carboxylate (VIId')

A mixture of VId' (500 mg, 0.53 mmole) and NaI (240 mg, 1.6 mmoles) in acetone (3 mL) was stirred for 2 hours at room temperature and then evaporated *in vacuo*. To the residue were added $CH_2Cl_2$ (20 mL) and water (10 mL). The organic layer was washed with 10% w/v sodium thiosulfate (5 mL) and aqueous NaCl (5 mL), dried over $MgSO_4$ and evaporated to dryness to give 540 mg (99%) of VIId' as an amorphous powder melting at 106°C (dec.).

IR: $\nu_{max}^{KBr}$ $cm^{-1}$ 3350, 1790, 1690.

UV: $\lambda_{max}^{EtOH}$ 240 ($E_{1\,cm}^{1\%}$ 270), 265 ($E_{1\,cm}^{1\%}$ 190).

NMR: $\delta^{CDCl_3}$ ppm 1.44 (9H, s), 1.65 (6H, s), 3.54 (2H, ABq), 4.28 (2H, s), 4.98 (1H, d, J=4.5Hz), 5.85 (1H, d-d, J=4.5 & 7.5Hz), 6.70 (1H, s), 6.90 (1H, s), 7.1—7.5 (25H, m).

Preparation No. 13

Diphenylmethyl 7-[(Z)-2-(1-t-Butoxycarbonylcyclobut-1-oxyimino)-2-(2-tritylaminothiazol-4-yl)acetamido]-3-chloromethyl-3-cephem-4-carboxylate (VIe')

Phosphorus pentachloride (1.46 g, 7 mmoles) was added to a suspension of (Z)-2-(1-t-butoxycarbonylcyclobut-1-oxyimine 2-(2-tritylaminothiazol-4-yl)acetic acid [IVe'] (4.09 g, 7 mmoles) in 70 mL of dry methylene chloride, and the mixture was stirred for 1 hour at room temperature. The acid chloride soloution was added at −20°C to a solution of silylated 7-ACA ester, which was prepared by adding BSA (5.6 mL, 21 mmoles) to a stirred suspension of benzhydryl 7-amino-3-chloromethyl-3-cephem-4-carboxylate hydrochloride [V] (3.16 g, 7 mmoles) in dry methylene chloride (70 mL). The mixture was stirred for 20 minutes at −10°C and then at room temperature for 40 minutes. The reaction mixture was evaporated and diluted with ethyl acetate (300 mL), and the organic layer was washed with 5% aqueous sodium bicarbonate, water and a saturated sodium chloride solution. After drying over sodium sulfate, the solvent was evaporated and the residue was purified by silica gel column chromatography (Wako gel C-200, 60 g); elution with chloroform. The fractions containing the desired product were combined and evaporated to obtain 5.88 g (86%) of VIe' as a yellow powder.

IR: $\nu_{max}^{KBr}$ $cm^{-1}$ 1790, 1725, 1525.

UV: $\lambda_{max}^{EtOH}$ nm 254 nm ($E_{1\,cm}^{1\%}$ = 214).

Preparation No. 14

Diphenylmethyl 7-[(Z)-2-(1-t-Butoxycarbonylcyclobut-1-oxyimino)-2-(2-tritylaminothiazol-4-yl)acetamido]-3-iodomethyl-3-cephem-4-carboxylate (VIIe')

To a stirred solution of VIe' (5.4 g, 5.5 mmoles) in acetone (108 mL) was added sodium iodide (2.48 g, 16.5 mmoles), and the mixture was stirred at room temperature for 3 hours. The reaction mixture was then filtered and evaporated to dryness, and the residue was dissolved in ethyl acetate (200 mL). The solution was washed with water (100 mL), 10% w/v sodium thiosulfate (40 mL) and saturated sodium chloride (3 × 70 mL). After drying over magnesium sulfate, the solvent was removed under reduced pressure to give 5.38 g (91%) of VIIe' as a yellow powder.

IR: $\nu_{max}^{KBr}$ $cm^{-1}$ 1790, 1725, 1690, 1525.

UV: $\lambda_{max}^{EtOH}$ nm 254 nm ($E_{1\,cm}^{1\%}$ = 184).

NMR: $\delta^{CTCl_3}$ ppm 1.45 (9H, s), 1.8—2.8 (6H, m), 3.52 (2H, ABq), 4.25 (2H, s), 4.98 (1H, d, J=5.3Hz), 5.87 (1H, dd, J=9 & 5.3Hz), 6.70 (1H, s), 6.88 (1H, s), 6.90 (1H, s), 7.28 (25H, s), 8.41 (1H, d, J=9Hz).

Example 1

7-[(Z)-2-(2-Aminothiazol-4-yl)-2-methoxyiminoacetamido]-3-{(5-thiazolo[4,5-c]pyridinio)methyl}-3-cephem-4-carboxylate (Ia)

A mixture of benzhydryl 3-iodomethyl-7-[(Z)-2-methoxyimino-2-(2-tritylaminothiazol-4-yl)acetamido]-3-cephem-4-carboxylate [VIIa] (1.50 g, 1.61 mmoles) and thiazolo[4,5-c]pyridine (300 mg, 2.20 mmoles) in dry dimethylsulfoxide (DMSO) (7.5 mL) was stirred at room temperature for 1 hour. The reaction mixture was diluted with ethyl acetate (20 mL), washed with water (5 mL), dried and evaporated to dryness. To a solution of the residue in $CHCl_3$ (5 mL) was added ether (50 mL) to give the quaternized blocked cephalosporin [XIIIa] (1.7 g), which was collected by filtration and treated with 90% trifluoroacetic acid [TFA] (17 mL) at room temperature for 1 hour. After evaporation of the solvent, the residue was triturated with ether, filtered and dissolved in $CH_3OH$ (2 mL). The solution was treated with 1M sodium 2-ethylhexanoate [SEH] in ethyl acetate (3.2 mL) at room temperature for 30 minutes to afford 774 mg of the crude title compound (Ia), which was purified by HPLC (Column, Lichrosorb RP-18, 8 × 300 mm; eluted with 0.01M ammonium phosphate buffer, pH 7.2—15% $CH_3OH$) and HP-20 column chromatography (3 × 30 cm, washed with 1L of water and eluted with 700 mL of 30% $CH_3OH$). The fractions containing the desired product were collected, concentrated and lyophilized to afford 180 mg (22%) of the title compound (Ia) as a colorless powder, mp 165°C (dec.).

IR: $\nu_{max}^{KBr}$ $cm^{-1}$ 3400, 1770, 1615.

UV: $\lambda_{max}^{phosphate\ buffer,\ pH\ 7}$ nm(ε) 225 (43400), 261 (21000).

NMR: $\delta^{D_2O}$ ppm 3.53 (2H, ABq), 4.03 (3H, s), 5.36 (1H, d, J=4.8Hz), 5.88 (1H, d), 6.94 (1H, s), 8.9 (2H, m), 9.81 (1H, s), 9.88 (1H, s).

Example 2

7-[(Z)-2-(2-Aminothiazol-4-yl)-2-methoxyiminoacetamido]-3-{(5-thiazolo[4,5-c]pyridinio)methyl}-3-cephem-4-carboxylate (Ia)

A mixture of benzhydryl 3-iodomethyl-7-[(Z)-2-methoxyimino-2-(2-tritylaminothiazol-4-yl)acetamido]-3-cephem-4-carboxylate [VIIa] (18.6 g, 0.02 mole) and thiazolo[4,5-c]pyridine (3.54 g, 0.026 mole) in 60 mL of dry DMSO was stirred at room temperature for one hour. The reaction mixture was diluted with 300 mL of chloroform, washed with 200 mL of water and dried over anhydrous $MgSO_4$. The chloroform solution was treated with active carbon (2 g) and filtered. The filtrate was concentrated to 30 mL and diluted with 1L of ether. The resulting precipitate was collected by filtration and washed with ether to give 23 g of the blocked cephalosporin [XIIIa], which was deblocked by stirring with 100 mL of 90% trifluoroacetic acid at room temperature for one hour. The mixture was concentrated to 10 mL below 30°C and poured into 1L of

ether. The resulting solid was collected by filtration and dissolved in 50 mL of methanol. Insolubles were removed by filtration. To the filtrate was added 40 mL (0.04 mole) of SEH in ethyl acetate with shaking and the mixture was diluted with 1 L of ethyl acetate. The precipitate which separated was collected by filtration, washed with ethyl acetate and dried *in vacuo* over $P_2O_5$ to give 11 g of crude title compound (Ia). The crude product was dissolved in 1500 mL of water and insoluble material (4.2 g) was removed by filtration. The filtrate was evaporated to dryness to give an oily residue which was dissolved in 300 mL of water at 30—35°C. The solution was filtered, concentrated to 50 mL and lyophilized to give an amorphous powder, which was dissolved in 30 mL of 5% methanol and chromatographed by preparative HPLC (Waters System 500, PrepPAK-500/$C_{18}$). Fractions containing the desired product were collected and concentrated to 30 mL and lyophilized to give 3.64 g (34%) of the purified title compound (Ia). Mp ca. 170°C (gradually dec.).

A more soluble form of Compound Ia was prepared as follows. A stirred mixture of Compound Ia prepared in the above procedure (250 mg) and distilled water (1.0 mL) was not completely soluble (indicated pH was 4.3). Powdered $NaHCO_3$ was added in small portions, with stirring. A clear solution was obtained after adding 5.0 mg of $NaHCO_3$ and the indicated pH then was 6.0. After filtration through a Millipore membrane filter to remove any insoluble impurities, the filtrate was lyophilized to give 245 mg of a water-soluble form of Compound Ia, mp 165°C. Estimated purity 80% (by HPLC). The sample was completely soluble in water to give a 25% solution.

IR: $\nu_{max}^{KBr}$ cm$^{-1}$ 3400, 1770, 1615.

UV: $\lambda_{max}^{phosphate\ buffer,\ pH\ 7}$ nm(ε) 225 (44200), 260 (21600).

| | | | |
|---|---|---|---|
| Anal. Calc'd for $C_{20}H_{17}N_7O_5S_3 \cdot 3H_2O$: | C, 41.02; | H, 3.96; | N, 16.74. |
| Found: | C, 40.92; | H, 3.18; | N, 16.61. |

## Example 3

7-[(Z)-2-(2-Aminothiazol-4-yl)-2-ethoxyiminoacetamido]-3-{(5-thiazolo[4,5-c]pyridinio)methyl}-3-cephem-4-carboxylate (Ib)

A mixture of benzhydryl 7-[(Z)-2-ethoxyimino-2-(2-tritylaminothiazol-4-yl)acetamido]-3-iodomethyl-3-cephem-4-carboxylate [VIIb] (517 mg, 0.5 mmole) and thiazolo[4,5-c]pyridine (88 mg, 0.65 mmole) in DMSO (5 mL) was stirred for 1 hour at room temperature. The reaction mixture was diluted with $CHCl_3$ (40 mL), washed with water and dried over $MgSO_4$. The filtrate was evaporated *in vacuo* and the residue was washed with ether and dissolved in 90% aqueous TFA. After standing for 1 hour at room temperature, the reaction mixture was concentrated *in vacuo*. The addition of ether to the concentrate resulted in separation of the product, which was collected by filtration and dissolved in a small amount of $CH_3OH$. The solution was chromatographed on an HP-20 column (50 mL) by eluting with 30% aqueous $CH_3OH$. The eluate was lyophilized to give 133 mg (47%) of the title compound (Ib) as an amorphous powder. Mp >160°C (dec.).

UV: $\lambda_{max}^{phosphate\ buffer,\ pH\ 7}$ nm(ε) 226 (44000), 258 (20000).

NMR: $\delta^{D_2O}$ ppm 1.38 (3H, t), 3.57 (2H, q), 4.32 (2H, q), 5.37 (1H, d), 5.70 (2H, d), 5.93 (1H, d), 6.98 (1H, s), 8.70—9.0 (2H, m), 9.80—9.92 (2H, m).

## Example 4

7-[(Z)-2-Allyloxyimino-2-(2-aminothiazol-4-yl)acetamido]-3-{(5-thiazolo[4,5-c]pyridinio)methyl}-3-cephem-4-carboxylate (Ic)

A mixture of benzhydryl 7-[(Z)-2-allyloxyimino-2-(2-tritylaminothiazol-4-yl)acetamido]-3-iodomethyl-3-cephem-4-carboxylate [VIIc] (478 mg, 0.5 mmole) and thiazolo[4,5-c]pyridine (108 mg, 0.8 mmole) in DMSO (1 mL) was stirred for 50 minutes at room temperature. The mixture was diluted with chloroform (100 mL), washed with water and evaporated under reduced pressure. The oily residue was triturated with ether and collected by filtration (610 mg). A mixture of this material and TFA (3 mL) was allowed to stand at room temperature for 2 hours and diluted with ether to precipitate the trifluoroacetate of the title compound (465 mg), which was collected by filtration and chromatographed on a column of HP-20 (1.8 × 15 cm). The column was eluted with water (1L) and 30% aqueous methanol (1L). The methanolic eluate was evaporated under reduced pressure and the residue was lyophilized to give the crude title compound (58 mg), which was purified by HPLC [Column, Lichrosorb RP-18; Mobile phase, 0.01M $NH_4H_2PO_4$ (pH 7):$CH_3OH$=75:25]. The eluate of the HPLC was chromatographed on a column of HP-20 (1.8 × 10 cm). The column was eluted with water (550 mL) and 50% aqueous methanol (500 mL). The methanolic eluate was evaporated under reduced pressure and the residue was lyophilized to give 31 mg (11%) of the title compound (Ic) as a pale yellow powder. Mp >160°C (dec.).

IR: $\nu_{max}^{KBr}$ cm$^{-1}$ 3600—3000, 1770, 1660, 1535.

UV: $\lambda_{max}^{phosphate\ buffer,\ pH\ 7}$ nm(ε) 225 (44000), 260 (21300).

NMR: $\delta^{D_2O + NaHCO}$ ppm 3.50 (1H, d, 18Hz, 2-H), 3.85 (1H, d, 18Hz, 2-H), 5.95 (1H, d, 4Hz, 7-H), 7.00 (1H, s, thiazole-H), 8.90 (2H, m, thiazolopyridine-H), 9.85 (1H, s, thiazolopyridine-H), 9.93 (1H, s, thiazolopyridine-H).

## Example 5

7-[(Z)-2-(2-Aminothiazol-4-yl)-2-(2-carboxyprop-2-oxyimino)-acetamido]-3-{(5-thiazolo[4,5-c]pyridinio)methyl}-3-cephem-4-carboxylate (Id)

To a stirred solution of diphenylmethyl 7-[(Z)-2-t-butoxycarbonylprop-2-oxyimino)-2-(2-tritylaminothiazol-4-yl)acetamido]-3-iodomethyl-3-cephem-4-carboxylate [VIId'] (700 mg, 0.66 mmole) in 3 mL of DMSO, was added thiazolo[4,5-c]pyridine (115 mg, 0.84 mmole) in two portions and the mixture was stirred for 1.25 hours at room temperature. The reaction mixture was diluted with ether (150 mL) to separate an oily product, which was washed again with ether. The residue was dissolved in methylene chloride (100 mL) and the solution was filtered and evaporated to give 655 mg of the blocked product [XIIId']. The yellow product (630 mg) was suspended in anisole (1 mL) and, with ice cooling, was treated with a mixture of TFA (10 mL) and water (0.5 mL). The mixture was stirred for 20 minutes with ice cooling and then for 1 hour at room temperature. After evaporation of the mixture below 30°C, the residue was triturated with isopropyl ether (50 mL). The precipitate was collected by filtration, and dried under reduced pressure to obtain 485 mg of crude title compound as a yellow powder. The crude product (480 mg) was dissolved in a small volume of aqueous sodium bicarbonate and purified by HPLC (Column, Lichrosorb RP-18; mobile phase, water). The desired fractions were combined (ca. 90 mL) and acidified to pH 2 with dilute hydrochloric acid. The acidified solution was passed through an HP-20 column (50 mL) and washed with water (200 mL). The fractions eluted with 40% methanol were combined, concentrated and lyophilized to afford 154 mg of pale yellow amorphous powder, which was slightly soluble in water. Microanalysis showed that this was the free acid of the title compound (Id). Mp >180°C (dec.).

IR: $\nu_{max}^{KBr}$ $cm^{-1}$ 1770, 1660, 1610, 1540

UV: $\lambda_{max}^{phosphate\ buffer,\ pH\ 7}$ nm(ε) 225 (45900), 260 (22200).

NMR: $\delta^{DMSO-d_6}$ ppm 1.40 (6H, s), 5.10 (1H, d, J=4Hz), 5.33 (1H, br, s), 5.70 (1H, br, s), 5.80 (1H, m), 6.66 (1H, s), 9.14 (2H, ABq), 9.88 (1H, s), 10.33 (1H, br, s).

Anal. Calc'd for $C_{23}H_{21}N_7O_7S_3 \cdot 2.5H_2O$: C, 42.59; H, 4.04; N, 15.11; S, 14.83.

Found: C, 42.69, 42.58; H, 3.49, 3.47; N, 15.15, 15.08; S, 15.01.

Atomic absorption analysis: Na, 0.01.

The free acid of Compound Id (100 mg) was dissolved in a small volume of aqueous sodium bicarbonate and subjected to HPLC (Column, Lichrosorb RP-18; mobile phase, water). The eluate containing the desired product which was concentrated and lyophilized to give 30 mg of the sodium salt of Id as a pale yellow amorphous powder, which was more than 25% soluble in water. Mp 178—196°C (dec.).

IR: $\nu_{max}^{KBr}$ $cm^{-1}$ 1770, 1605, 1540

UV: $\lambda_{max}^{phosphate\ buffer,\ pH\ 7}$ nm(ε) 225 (42400), 260 (21000).

NMR: $\delta^{D_2O}$ ppm 1.57 (6H, s), 3.55 (2H, ABq), 5.38 (1H, d, J=4.5Hz), 5.70 (2H, ABq), 5.93 (1H, d, J=4.5Hz), 6.94 (1H, s), 8.89 (2H, ABq), 9.83 (1H, s), 9.92 (1H, s).

Anal. Calc'd for $C_{23}H_{20}N_7O_7S_3Na \cdot 3H_2O$: C, 40.64; H, 3.86; N, 14.43; S, 14.15; Na, 3.38.

Found: C, 40.54, 40.65; H, 3.52, 3.48; N, 14.35, 14.41; S, 14.16; Na, 3.35.

## Example 6

7-[(Z)-2-(2-Aminothiazol-4-yl)-2-(2-carboxyprop-2-oxyimino)acetamido]-3-{(5-thiazolo[4,5-c]pyridinio)methyl}-3-cephem-4-carboxylate (Id)

A solution of diphenylmethyl 7-[(Z)-(2-t-butoxycarbonylprop--2-oxyimino)-2-(2-tritylaminothiazol-4-yl)acetamido]-3-iodomethyl-3-cephem-4-carboxylate [VIId'] (9.0 g, 8.5 mmoles) and thiazolo[4,5-c]pyridine (1.5 g, 11 mmoles) in dimethylsulfoxide (38 mL) was stirred at room temperature for 1 hour. The reaction mixture was poured into ether (500 mL) and, after decantation, the oily precipitate was dissolved in chloroform (300 mL). The solution was washed with water (2 × 250 mL), dried over $Na_2SO_4$ and evaporated *in vacuo*. The oily residue was triturated with ether to give a yellow solid (8.5 g) which was treated with a mixture of anisole (4.3 mL), water (2.2 mL) and TFA (43 mL) at 0°C for 5 minutes and then at room temperature for 1 hour. The reaction mixture was evaporated and the oily residue was triturated with isopropyl ether to give 7.49 g of the crude title product (estimated purity 42%). The product was dissolved in aqueous $NaHCO_3$ and purified by HPLC (Waters System 500, prepPack-S-500/$C_{18}$, mobile phase $H_2O$), yielding 1.55 g (36%) of the title compound (Id) as its Na salt, which was more than 25% soluble in water. Mp >180°C (dec.).

IR: $\nu_{max}^{KBr}$ $cm^{-1}$ 3400, 1770, 1660, 1610, 1540, 1480, 1400, 1360, 1190, 1150, 1120, 1085, 1040, 970, 920, 840, 790, 760.

UV: $\lambda_{max}^{phosphate\ buffer,\ pH\ 7}$ nm(ε) 225 (45700), 260 (22000).

NMR: $\delta^{D_2O}$ ppm 1.47 (6H, s), 3.20 & 3.70 (1H each, d, J=18Hz), 5.26 (1H, d, J=4.5Hz), 5.40 & 5.75 (1H each, d, J=15Hz), 5.78 (1H, d, J=4.5Hz), 6.68 (1H, s), 8.68 (1H, d, J=7.5Hz), 8.88 (1H, d, J=7.5Hz), 9.68 (1H, s), 9.84 (1H, s).

## Example 7

7-[(Z)-2-(2-Aminothiazol-4-yl)-2-(1-carboxycyclobut-1-oxyimino)acetamido]-3-{(5-thiazolo[4,5-c]pyridinio)methyl}-3-cephem-4-carboxylate (Ie)

To a solution of diphenylmethyl 7-[(Z)-2-(1-t-butoxycarbonylcyclobut-1-oxyimino)-2-(2-tritylaminothiazol-4-yl)acetamido]-3-iodomethyl-3-cephem-4-carboxylate (VIIe'] (482 mg, 0.45 mmole) in 2 mL of DMSO was added thiazolo[4,5-c]pyridine (68 mg, 0.5 mmole) in one portion. The mixture was stirred for 45 minutes at room temperature. The reaction mixture was then diluted with ether (50 mL) and the precipitated oil was triturated in ether (4 × 50 mL). The precipitate was mixed with anisole (0.5 mL),

trifluoroacetic acid (5 mL) and water (0.5 mL), and the mixture was stirred with ice cooling for 30 minutes and at room temperature for 1 hour, and then was concentrated under reduced pressure (<30°C). The residue was triturated with isopropyl ether (100 mL), and the resulting precipitate was collected by filtration to afford 395 mg of crude title compound, as a yellow powder. The crude product (387 mg) was purified on an HP-20 column (100 mL). The eluate containing desired product, which was eluted with 50% methanol, was concentrated and lyophilized to give 80 mg (31%) of the title product (Ie) as a pale yellow amorphous powder. Mp >170°C (dec.).

IR: $\nu_{max}^{KBr}$ $cm^{-1}$ 1775, 1663, 1620, 1540.

UV: $\lambda_{max}^{phosphate\ buffer,\ pH\ 7}$ nm(ε) 224.5 (46000), 259 (22500).

NMR: $\delta^{D_2O(NaHCO_3)}$ ppm 1.8—2.7 (6H, m), 3.56 (2H, ABq), 5.42 (1H, d, J=4.5Hz), 5.70 (2H, ABq), 5.94 (1H, d, J=4.5Hz), 6.93 (1H, s), 8.90 (2H, ABq), 9.82 (1H, s), 9.94 (1H, s).

Example 8

7-[(Z)-2-(2-Aminothiazol-4-yl)-2-methoxyiminoacetamido]-3-[{5-(2-aminothiazolol[4,5-c]pyridinio)}methyl]-3-cephem-4-carboxylate (If)

To a stirred solution of benzhydryl 3-iodomethyl-7-[(Z)-2-methoxyimino-2-(2-tritylaminothiazol-4-yl)acetamido]-3-cephem-4-carboxylate [VIIa] (466 mg, 0.5 mmole) in 3 mL of DMSO, was added 2-aminothiazolo[4,5-c]pyridine (100 mg, 0.66 mmole). The mixture was stirred at room temperature for 1.2 hours and diluted with ether (50 mL). The deposited oil was triturated with ether and the solvent was decanted. This procedure was repeated another three times. A solution of the residual precipitate in 50 mL of $CHCl_3$—$CH_3OH$ (4:1) was filtered and concentrated *in vacuo*. The residue was triturated in isopropyl ether (50 mL). The precipitate (602 mg) was collected by filtration and treated with anisole (0.5 mL) and 90% TFA with cooling in an ice-water bath. After one hour stirring at room temperature, the mixture was evaporated *in vacuo* and the residue was triturated with isopropyl ether (50 mL). The precipitate was collected by filtration to give 414 mg of crude If as a tan powder, which was purified by column chromatograhy on HP-20 resin (100 mL), eluting with water and 30% $CH_3OH$ successively. The desired fractions which were eluted with 30% $CH_3OH$, were combined, concentrated and lyophilized to obtain 36 mg (13%) of the title compound (If) as a pale yellow amorphous powder. Estimated purity 60% (by HPLC).

IR: $\nu_{max}^{KBr}$ $cm^{-1}$ 1770, 1640, 1610, 1540.

UV: $\lambda_{max}^{phosphate\ buffer,\ pH\ 7}$ nm(ε) 246 (43600).

NMR: $\delta^{DMSO-d_6(D_2O)}$ ppm 3.78 (3H, s), 5.07 (1H, d, J=4), 5.67 (1H, d, J=4), 6.67 (1H, s), 8.57 (2H, ABq), 9.25 (1H, s).

Example 9

7-[(Z)-2-(2-Aminothiazol-4-yl)-2-(2-carboxyprop-2-oxyimino)acetamido]-3-[{5-(2-aminothiazolo[4,5-c]pyridinio)}methyl]-3-cephem-4-carboxylate (Ig)

A mixture of diphenylmethyl 7-[(Z)-2-t-butoxycarbonylprop-2-oxyimino)-2-(2-tritylaminothiazol-4-yl)acetamido]-3-iodomethyl-3-cephem-4-carboxylate ([VIId'] (455 mg, 0.43 mmole) and 2-aminothiazolo[4,5-c]pyridine (75 mg, 0.5 mmole) in 2.5 mL of DMSO was stirred for 1.5 hours at room temperature and then treated with ether (50 mL) to separate an oily precipitate, which was triturated with ether. The ether layer was removed by decantation. This procedure was repeated another three times. A solution of the residual precipitate in 30 mL of $CHCl_3$—$CH_3OH$ (4:1) was filtered, the filtrate was evaporated and the residue was triturated with isopropyl ether. The precipitate (349 mg) was collected by filtration and treated with anisole (0.3 mL) and 90% TFA (3 mL) with ice cooling. The mixture was stirred at room temperature for 1.5 hours and evaporated *in vacuo*, and the residue was triturated with isopropyl ether (50 mL) to give 280 mg of crude Ig as a yellow powder, which was purified by HP-20 resin column chromatography (40 mL). The column was eluted with water and subsequently with 30% aqueous $CH_3OH$. The desired fractions, eluted with 30% $CH_3OH$, were combined, concentrated and lyophilized to afford 19 mg (7%) of the title compound (Ig) as a pale yellow amorphous powder. Estimated purity 60% (by HPLC).

IR: $\nu_{max}^{KBr}$ $cm^{-1}$ 1770, 1660sh, 1635, 1540.

UV: $\lambda_{max}^{phosphate\ buffer,\ pH\ 7}$ nm(ε) 247 (41700).

NMR: $\delta^{DMSO-d_6(D_2O)}$ ppm 1.42 (6H, s), 5.10 (1H, d, J=4), 5.75 (1H, d, J=4, 6.66 (1H, s), 8.54 (2H, ABq), 9.23 (1H, s).

Example 10

7-[(Z)-2-(2-Aminothiazol-4-yl)-2-methoxyiminoacetamido]-3-[{5-(2-methylthiazolo[4,5-c]pyridinio)}methyl]-3-cephem-4-carboxylate (Ih)

A mixture of benzhydryl 3-iodomethyl-7-[(Z)-2-methoxyimino-2-(2-tritylaminothiazol-4-yl)acetamido]-3-cephem-4-carboxylate [VIIa] (466 mg, 0.5 mmole) and 2-methylthiazolo[4,5-c]pyridine (100 mg, 0.67 mmole) in 2.5 mL of DMSO was stirred at room temperature for an hour and diluted with ether (150 mL). The resulting oil was triturated with ether and the solvent was decanted. This procedure was repeated another three times. A solution of the residual precipitate in 50 mL of $CHCl_3$—$CH_3OH$ (4:1) was filtered and evaporated, and the residue was triturated with isopropyl ether (50 mL) to afford 558 mg of the quaternized blocked cephalosporin ($\nu^{KBr}$ $cm^{-1}$: 1785, 1720, 1670, 1625, 1525, 1030), which was treated with anisole (0.5 mL) and 90% TFA (5 mL) with cooling in an ice-water bath. The mixture was stirred at room temperature for

1.5 hours and evaporated below 40°C. The residue was triturated with isopropyl ether (30 mL) to give 395 mg of a yellow powder. The crude powder was dissolved in a mixture of 98% formic acid (2 mL) and concentrated hydrochloric acid (0.1 mL), and the solution was allowed to stand at room temperature for 30 minutes. The resulting mixture was chromatographed on a column of HP-20 resin (100 mL) by eluting with water and 30% $CH_3OH$ successively. The fractions containing the desired product were combined, concentrated and lyophilized to give 161 mg (59%) of the title compound (Ih) as a yellow amorphous powder. Estimated purity 65% (by HPLC).

IR: $\nu_{max}^{KBr}$ $cm^{-1}$ 1770, 1660, 1620, 1535.

UV: $\lambda_{max}^{phosphate\ buffer,\ pH\ 7}$ nm(ε) 228 (50300), 260 (22000).

NMR: $\delta^{D_2O}$ ppm 3.06 (3H, s), 3.51 (2H, ABq), 4.04 (3H, s), 5.36 (1H, d, J=4), 5.61 (2H, ABq), 5.84 (1H, d, J=4), 6.75 (1H, s), 8.72 (2H, ABq), 9.68 (1H, s).

Example 11

7-[(Z)-2-(2-Aminothiazol-4-yl)-2-methoxyiminoacetamido]-3-[{5-(2-methylthiothiazolo[4,5-c]pyridinio)}methyl]-3-cephem-4-carboxylate (Ii)

A mixture of benzhydryl 3-iodomethyl-7-[(Z)-2-methoxyimino-2-(2-tritylaminothiazol-4-yl)acetamido]-3-cephem-4-carboxylate [VIIa] (470 mg, 0.51 mmole) and 2-methylthiothiazolo[4,5-c]pyridine (121 mg, 0.66 mmole) in 3 mL of dry DMSO was allowed to stand for 30 minutes at room temperature. The reaction mixture was diluted with ethyl acetate (30 mL), washed with water (3 × 20 mL) and aqueous NaCl, dried over anhydrous $Na_2SO_4$ and concentrated under reduced pressure. The residue was treated with ethyl ether and filtered to give 475 mg of the quaternized blocked cephalosporin, which was dissolved in 3 mL of TFA (99%) and allowed to stand for 1.5 hours at ambient temperature. Removal of the solvent followed by trituration with isopropyl ether (30 mL) gave 360 mg of the TFA salt of Ii as a powder. The TFA salt was chromatographed on a column of HP-20 resin and eluted with water (300 mL), 10% $CH_3OH—H_2O$ (300 mL) and 30% $CH_3OH—H_2O$ (300 mL), successively. Fractions eluted with 30% $CH_3OH—H_2O$ were combined, concentrated *in vacuo* and lyophilized to afford 136 mg (25%) of the title compound (Ii). Estimated purity 50% (by HPLC).

IR: $\nu_{max}^{KBr}$ $cm^{-1}$ 1770, 1660, 1615, 1530.

UV: $\lambda_{max}^{phosphate\ buffer,\ pH\ 7}$ nm(ε) 262 (36900), 294 (16500).

NMR: $\delta^{D_2O}$ ppm 2.91 (3H, s, —$SCH_3$), 3.50 (2H, m, 2-$CH_2$), 4.09 (3H, s, =N—$OC\underline{H_3}$), 5.37 (1H, d, J=5, 6-H), 5.71 (2H, ABq, 3-$CH_2$), 5.87 (1H, d, J=5, 7-H), 6.86 (1H, s, thiazole-5-H), 8.55 (1H, d, J=6, thiazolopyridinio-7'-H), 8.81 (1H, d, J=6, thiazolopyridinio-6'-H), 9.50 (1H, s, thiazolopyridinio-4'-H).

Example 12

7-[(Z)-2-(2-Aminothiazol-4-yl)-2-ethoxyiminoacetamido]-3-[{5-(2-aminothiazolo[4,5-c]pyridinio)}methyl]-3-cephem-4-carboxylate (Ij)

To a stirred solution of benzhydryl 7-[(Z)-2-ethoxyimino-2-(2-tritylaminothiazol-4-yl)acetamido]-3-iodomethyl-3-cephem-4-carboxylate [VIIb] (600 mg, 0.64 mmole) in 4 mL of DMSO, was added 2-aminothiazolo[4,5-c]pyridine (128 mg, 0.85 mmole). The mixture was stirred at room temperature for 40 minutes and then diluted with ether (100 mL). The oily precipitate which separated was triturated with ethyl ether and the solvent was decanted. This procedure was repeated another four times. A solution of the residual precipitate in 50 mL of $CHCl_3—CH_3OH$ (4:1) was filtered and the filtrate was concentrated to give an oily residue, which was triturated with isopropyl ether (50 mL). The solid thus obtained was collected by filtration to give 675 mg of the quaternized blocked cephalosporin as yellow powder. The powder was treated with anisole (2 mL) and 90% TFA (11 mL) with ice cooling. The mixture was stirred at room temperature for 1.5 hours and evaporated *in vacuo*. The residue was triturated with isopropyl ether (50 mL) and collected by filtration to give 490 mg of crude Ij, which was purified by column chromatography using HP-20 resin (100 mL). The column was eluted with water and 30% aqueous methanol successively. The desired fractions which were obtained by elution with 30% $CH_3OH$ were combined, concentrated and lyophilized to afford 44 mg (13%) of the title compound (Ij) as a yellow amorphous powder. Estimated purity 30% (by HPLC).

IR: $\nu_{max}^{KBr}$ $cm^{-1}$ 1750, 1655sh, 1600, 1520.

UV: $\lambda_{max}^{phosphate\ buffer,\ pH\ 7}$ nm(ε) 246 (26800).

**Claims for the Contracting States: BE CH DE FR GB IT LI LU NL SE**

1. Compounds of the general formula I:

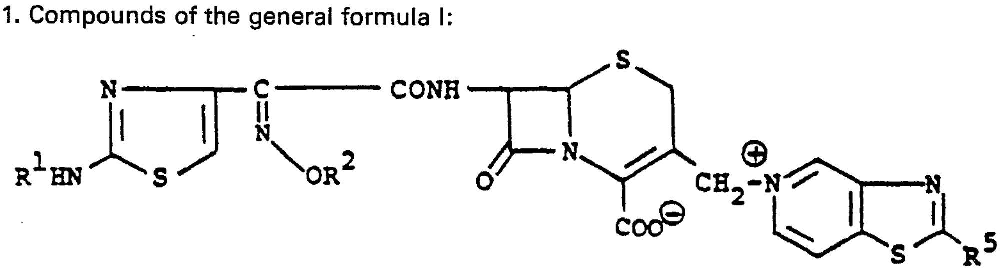

I

wherein $R^1$ is hydrogen or a conventional amino-protecting group, $R^2$ is a straight or branched chain alkyl group containing from 1 to 4 carbon atoms, allyl, 2-butenyl, 3-butenyl or a group of the formula

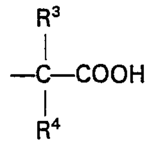

in which $R^3$ and $R^4$ each are independently hydrogen, methyl or ethyl, or $R^3$ and $R^4$, taken together with the carbon atom to which they are attached, may be a cycloalkylidene ring containing from 3 to 5 carbon atoms, and $R^5$ is hydrogen, amino, $C_1$—$C_6$-alkyl, $C_1$—$C_6$-alkoxy or $C_1$—$C_6$-alkylthio, or a nontoxic pharmaceutically acceptable salt, physiologically hydrolyzable ester or solvate thereof.

2. Compounds of Claim 1 wherein $R^1$ is hydrogen and $R^2$ is methyl, ethyl, allyl, 2-carboxyprop-2-yl, 1-carboxycycloprop-1-ylidene or 1-carboxycyclobut-1-ylidene, and $R^5$ is hydrogen, amino, methyl or methylthio; or a nontoxic pharmaceutically acceptable salt, physiologically hydrolyzable ester or solvate thereof.

3. Compounds of Claim 1 namely:

7-[(Z)-2-(2-aminothiazol-4-yl)-2-methoxyiminoacetamido]-3-{(5-thiazolo[4,5-c]pyridinio)methyl}-3-cephem-4-carboxylate;

7-[(Z)-2-(2-aminothiazol-4-yl)-2-ethoxyiminoacetamido]-3-{(5-thiazolo[4,5-c]pyridinio)methyl}-3-cephem-4-carboxylate,

7-[(Z)-2-(2-allyloxyimino)-2-(2-aminothiazol-4-yl)acetamido]-3-{(5-thiazolo[4,5-c]pyridinio)methyl}-3-cephem-4-carboxylate;

7-[(Z)-2-(2-aminothiazol-4-yl)-2-(2-carboxyprop-2-oxyimino)-acetamido]-3-{(5-thiazolo[4,5-c]pyridinio)methyl}-3-cephem-4-carboxylate;

7-[(Z)-2-(2-aminothiazol-4-yl)-2-(1-carboxycyclobut-1-oxyimino)-acetamido]-3-{(5-thiazolo[4,5-c]pyridinio)methyl}-3-cephem-4-carboxylate;

7-[(Z)-2-(2-aminothiazol-4-yl)-2-methoxyiminoacetamido]-3-[{5-(2-aminothiazolo[4,5-c]pyridinio)}methyl]-3-cephem-4-carboxylate;

7-[(Z)-2-(2-aminothiazol-4-yl)-2-ethoxyiminoacetamido]-3-[{5-(2-aminothiazolo[4,5-c]pyridinio)}methyl]-3-cephem-4-carboxylate;

7-[(Z)-2-(2-aminothiazol-4-yl)-2-(2-carboxyprop-2-oxyimino)acetamido]-3-[{(5-2-aminothiazolo[4,5-c]pyridinio)}methyl]-3-cephem-4-carboxylate;

7-[(Z)-2-(2-aminothiazol-4-yl)-2-methoxyiminoacetamido-3-[{5-(2-methylthiothiazolo[4,5-c]pyridinio)}methyl]-3-cephem-4-carboxylate; and

7-[(Z)-2-(2-aminothiazol-4-yl)-2-methoxyiminoacetamido-3-[{5-(2-methylthiazolo[4,5-c]pyridinio)}methyl]-3-cephem-4-carboxylate;

or a nontoxic pharmaceutically acceptable salt, physiologically hydrolyzable ester or solvate thereof.

4. Pharmaceutical composition comprising a pharmaceutically effective amount of at least one compound of Claims 1 to 3 and an inert pharmaceutical carrier.

5. Composition according to Claim 4, wherein the compound of Claims 1 to 3 is selected from the group consisting of:

a) 7-[(Z)-2-(2-aminothiazol-4-yl)-2-methoxyimino-acetamido]-3-{(5-thiazolo[4,5-c]pyridinio)methyl}-3-cephem-4-carboxylate,

b) 7-[(Z)-2-(2-aminothiazol-4-yl)-2-(2-carboxyprop-2-oxyimino)acetamido]-3-{(5-thiazolo[4,5-c]pyridinio)methyl}-3-cephem-4-carboxylate,

c) 7-[(Z)-2-(2-aminothiazol-4-yl)-2-ethoxyiminoacetamido]-3-{(5-thiazolo[4,5-c]pyridinio)methyl}-3-cephem-4-carboxylate,

d) 7-[(Z)-2-(2-aminothiazol-4-yl)-2-(1-carboxycyclobut-1-oxyimino)acetamido]-3-{(5-thiazolo[4,5-c]pyridinio)methyl}-3-cephem-4-carboxylate,

e) 7-[(Z)-2-(2-aminothiazol-4-yl)-2-methoxyiminoacetamido]-3-[{(5-(2-aminothiazolo[4,5-c]pyridinio)}methyl]-3-cephem-4-carboxylate,

f) 7-[(Z)-2-(2-aminothiazol-4-yl)-2-methoxyiminoacetamido]-3-[{5-(2-methylthiazolo[4,5-c]pyridinio)}methyl-3-cephem-4-carboxylate,

and the nontoxic pharmaceutically acceptable salts, physiologically hydrolyzable esters and solvates of said compounds.

6. An antibacterial composition in unit dosage form comprising from about 50 mg to about 1500 mg of at least one compound of Claims 1 to 3 and an inert pharmaceutical carrier.

7. Composition according to Claim 6 wherein the compound of Claims 1 to 3 is selected from the group as stated in claim 5.

8. Process for the manufacture of the compounds of Claims 1 to 3, characterized in that a compound of the general formula II:

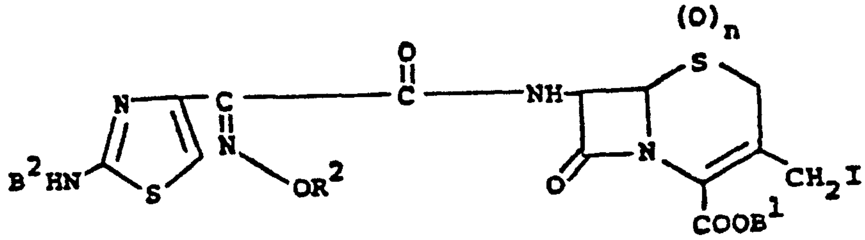

II

in which $R^2$ is as defined in Claim 1, $B^1$ is a conventional carboxyl-protecting, $B^2$ is a conventional amino-protecting group, n is 0 or 1, is reacted with a thiazolo[4,5-c]pyridine of the formula III:

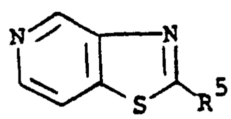

III

in which $R^5$ is as defined in Claim 1, to produce a compound of the formula IV:

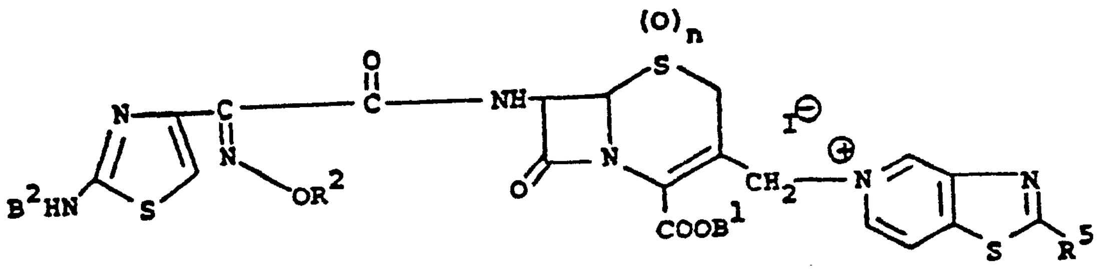

IV

in which $R^2$, $R^5$, $B^1$, $B^2$ and n are as defined above and,
if n is 1, the sulfoxide is reduced by conventional means, and subsequently all protecting groups are removed by conventional means to give compounds of formula I, wherein $R^1$ is hydrogen or the protecting group $B^1$ is removed to give compounds of formula I, wherein $R^1$ is a conventional amino-protecting group and/or if desired compounds of formula I are converted to their nontoxic pharmaceutically acceptable salts, physiologically hydrolyzable esters and solvates thereof.

9. Process for the manufacture of compounds of Claims 1 to 3, characterized in that a compound of the formula V:

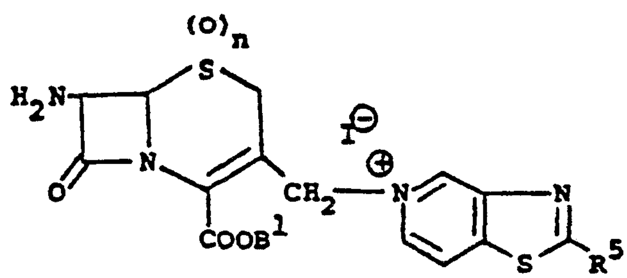

V

or an N-silyl derivative thereof, in which $B^1$ is hydrogen or a conventional carboxyl-protecting group, n is 0 or 1 and $R^5$ is as defined in Claim 1, is acylated with an acylating derivative of an acid of the formula VI:

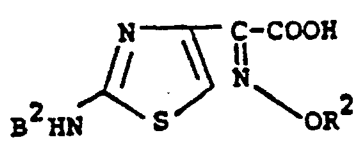

VI

in which $R^2$ is as defined in Claim 1 and any carboxyl group in $R^2$ is protected by a conventional protecting group $B^3$ and $B^2$ is a conventional amino-protecting group, to produce a compound of the formula VIII:

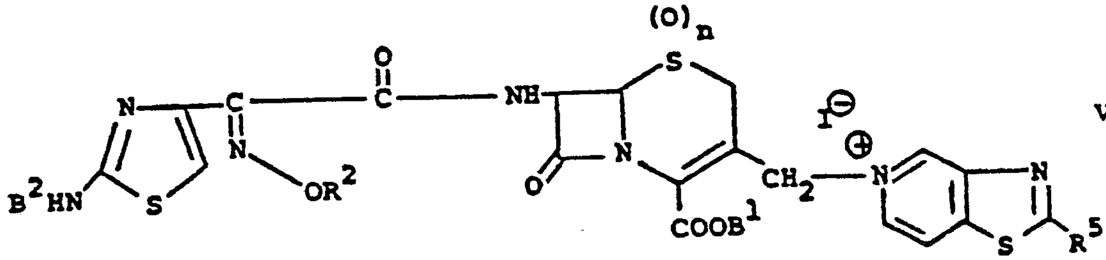

VIII

in which $R^2$, $R^5$, $B^1$, $B^2$ and n are as defined above and, if n is 1, the sulfoxide is reduced by conventional means, and subsequently all protecting groups are removed to produce compounds of formula I, wherein $R^1$ is hydrogen or the protecting groups $B^1$ and $B^3$ are removed to give compounds of formula I, wherein $R^1$ is a conventional amino-protecting group and/or if desired the compounds of formula I are converted to their nontoxic pharmaceutically acceptable salt, physiologically hydrolyzable esters and solvates thereof.

**Claims for the Contracting State: AT**

1. A process for the manufacture of the compounds of the general formula I:

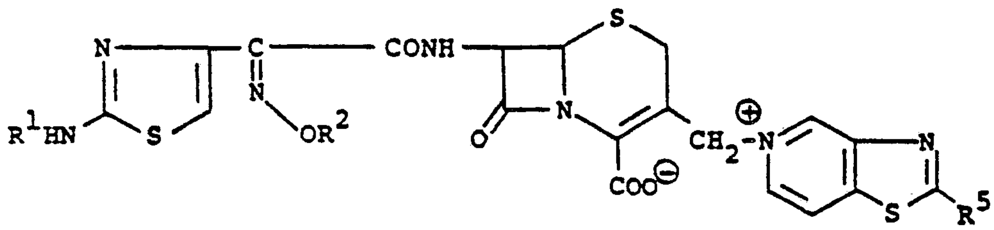

I

wherein $R^1$ is hydrogen or a conventional amino-protecting group, $R^2$ is a straight or branched chain alkyl group containing from 1 to 4 carbon atoms, allyl, 2-butenyl, 3-butenyl or a group of the formula

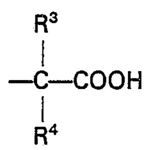

in which $R^3$ and $R^4$ each are independently hydrogen, methyl or ethyl, or $R^3$ and $R^4$, taken together with the carbon atom to which they are attached, may be a cycloalkylidene ring containing from 3 to 5 carbon atoms, and $R^5$ is hydrogen, amino, $C_1$—$C_6$-alkyl, $C_1$—$C_6$-alkoxy or $C_1$—$C_6$-alkylthio, or a nontoxic pharmaceutically acceptable salt, physiologically hydrolyzable ester or solvate thereof, characterized in that

A) a compound of the general formula II:

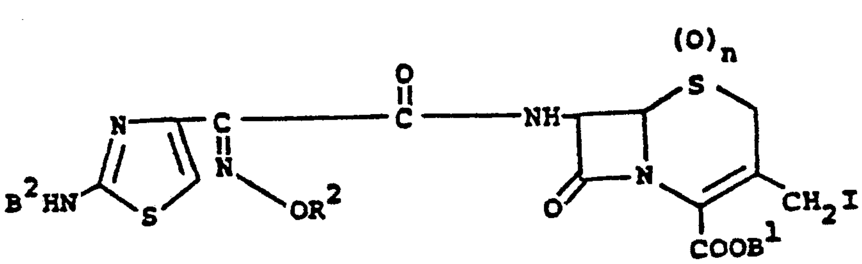

II

in which $R^2$ is as defined above, $B^1$ is a conventional carboxyl-protecting, $B^2$ is a conventional amino-protecting group, n is 0 or 1, is reacted with a thiazolo[4,5-c]pyridine of the formula III:

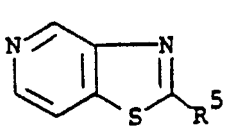

III

in which $R^5$ is as defined above to produce a compound of the formula IV:

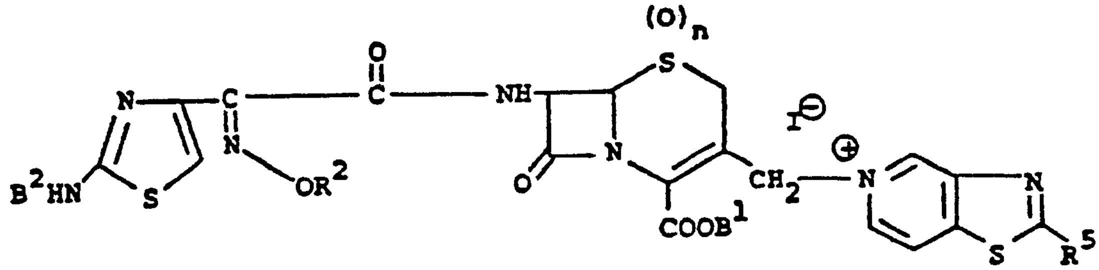

IV

in which $R^2$, $R^5$, $B^1$, $B^2$ and n are as defined above and, if n is 1, the sulfoxide is reduced by conventional means, and subsequently all protecting groups are removed by conventional means to give compounds of formula I, wherein $R^1$ is hydrogen or the protecting group $B^1$ is removed to give compounds of formula I, wherein $R^1$ is a conventional amino-protecting group and/or if desired compounds of formula I are converted to their nontoxic pharmaceutically acceptable salts, physiologically hydrolyzable esters and solvates thereof, or

B) a compound of the formula V:

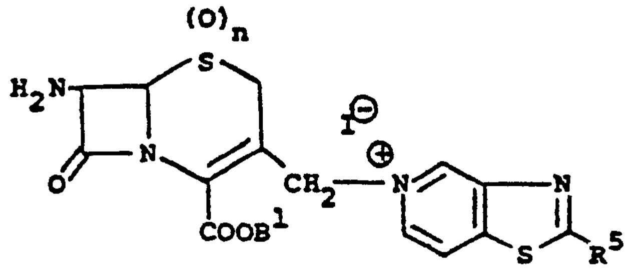

V

or an N-silyl derivative thereof, in which $B^1$ is hydrogen or a conventional carboxyl-protecting group, n is 0 or 1 and $R^5$ is as defined above is acylated with an acylating derivative of an acid of the formula VI:

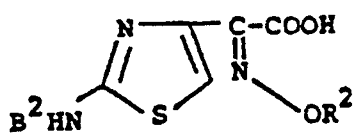

VI

in which $R^2$ is as defined above and any carboxyl group in $R^2$ is protected by a conventional protecting group $B^3$ and $B^2$ is a conventional amino-protecting group, to produce a compound of the formula VIII:

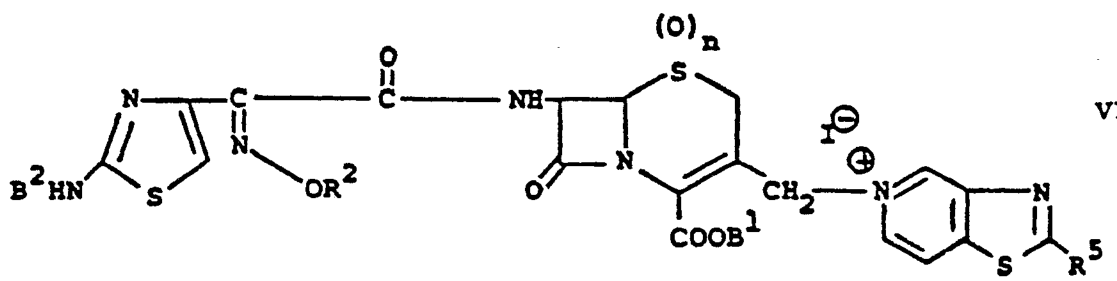

VIII

in which $R^2$, $R^5$, $B^1$, $B^2$ and n are as defined above and, if n is 1, the sulfoxide is reduced by conventional means, and subsequently all protecting groups are removed to produce compounds of formula I, wherein $R^1$ is hydrogen or the protecting group $B^1$ and $B^3$ are removed to give compounds of formula I, wherein $R^1$ is a conventional amino-protecting group and/or if desired the compounds of formula I are converted to their nontoxic pharmaceutically acceptable salt, physiologically hydrolyzable esters and solvates thereof.

2. The process of Claim 1, characterized in that compounds of formula I are obtained wherein $R^1$ is hydrogen and $R^2$ is methyl, ethyl, allyl, 2-carboxyprop-2-yl, 1-carboxycycloprop-1-ylidene or 1-carboxycyclobut-1-ylidene, and $R^5$ is hydrogen, amino, methyl or methylthio; or a nontoxic pharmaceutically acceptable salt, physiologically hydrolyzable ester or solvate thereof.

3. The process of Claim 1 characterized in that the following compounds are obtained:

7-[(Z)-2-(2-aminothiazol-4-yl)-2-methoxyiminoacetamido]-3-{(5-thiazolo[4,5-c]pyridinio)methyl}-3-cephem-4-carboxylate;

7-[(Z)-2-(2-aminothiazol-4-yl)-2-ethoxyiminoacetamido]-3-{(5-thiazolo[4,5-c]pyridinio)methyl}-3-cephem-4-carboxylate,

7-[(Z)-2-(2-allyloxyimino-2-(2-aminothiazol-4-yl)acetamido]-3-{(5-thiazolo[4,5-c]pyridinio)methyl}-3-cephem-4-carboxylate;

7-[(Z)-2-(2-aminothiazol-4-yl)-2-(2-carboxyprop-2-oxyimino)-acetamido]-3-{(5-thiazolo[4,5-c]pyridinio)methyl}-3-cephem-4-carboxylate;

7-[(Z)-2-(2-aminothiazol-4-yl)-2-(1-carboxycyclobut-1-oxyimino)-acetamido]-3-[{(5-thiazolo[4,5-c]pyridinio)methyl}-3-cephem-4-carboxylate;

7-[(Z)-2-(2-aminothiazol-4-yl)-2-methoxyiminoacetamido]-3-[{5-(2-aminothiazolo[4,5-c]pyridinio)}methyl]-3-cephem-4-carboxylate;

7-[(Z)-2-(2-aminothiazol-4-yl)-2-ethoxyiminoacetamido]-3-[{5-(2-aminothiazolo[4,5-c]pyridinio)}methyl]-3-cephem-4-carboxylate;

7-[(Z)-2-(2-aminothiazol-4-yl)-2-(2-carboxyprop-2-oxyimino)acetamido)-3-[{5-2-aminothiazolo[4,5-c]pyridinio)}methyl]-3-cephem-4-carboxylate;

7-[(Z)-2-(2-aminothiazol-4-yl)-2-methoxyiminoacetamido-3-[{5-(2-methylthiothiazolo[4,5-c]pyridinio)}methyl]-3-cephem-4-carboxylate; and

7-[(Z)-2-(2-aminothiazol-4-yl)-2-methoxyiminoacetamido-3-[{5-(2-methylthiazolo[4,5-c]pyridinio)}methyl]-3-cephem-4-carboxylate;

or a nontoxic pharmaceutically acceptable salt, physiologically hydrolyzable ester or solvate thereof.

4. A process for preparing a pharmaceutical composition characterized in that a pharmaceutically effective amount of at least one compound obtainable according to Claims 1 to 3 is incorporated into an inert pharmaceutical carrier.

5. The process of Claim 4, wherein the compound of Claims 1 to 3 is selected from the group consisting of:

a) 7-[(Z)-2-(2-aminothiazol-4-yl)-2-methoxyimino-acetamido]-3-{(5-thiazolo[4,5-c]pyridinio)methyl}-3-cephem-4-carboxylate,

b) 7-[(Z)-2-(2-aminothiazol-4-yl)-2-(2-carboxyprop-2-oxyimino)acetamido]-3-{(5-thiazolo[4,5-c]pyridinio)methyl}-3-cephem-4-carboxylate,

c) 7-[(Z)-2-(2-aminothiazol-4-yl)-2-ethoxyiminoacetamido]-3-{(5-thiazolo[4,5-c]pyridinio)methyl}-3-cephem-4-carboxylate,

d) 7-[(Z)-2-(2-aminothiazol-4-yl)-2-(1-carboxycyclobut-1-oxyimino)acetamido]-3-{(5-thiazolo[4,5-c]pyridinio)methyl}-3-cephem-4-carboxylate,

e) 7-[(Z)-2-(2-aminothiazol-4-yl)-2-methoxyiminoacetamido]-3-[{5-(2-aminothiazolo[4,5-c]pyridinio)}methyl]-3-cephem-4-carboxylate,

f) 7-[(Z)-2-(2-aminothiazol-4-yl)-2-methoxyiminoacetamido]-3-[{5-(2-methylthiazolo[4,5-c]pyridinio)}methyl-3-cephem-4-carboxylate,

and the nontoxic pharmaceutically acceptable salts, physiologically hydrolyzable esters and solvates of said compounds.

6. A process for preparing an antibacterial composition in unit dosage form characterized in that from about 50 mg to about 1500 mg of at least one compound obtainable according to Claims 1 to 3 are incorporated into an inert pharmaceutical carrier.

7. The process of Claim 6 wherein the compound of Claims 1 to 3 is selected from the group as stated in Claim 5.

**Patentansprüche für die Vertragsstaaten: BE CH DE FR GB IT LI LU NL SE**

1. Verbindungen der allgemeinen Formel I:

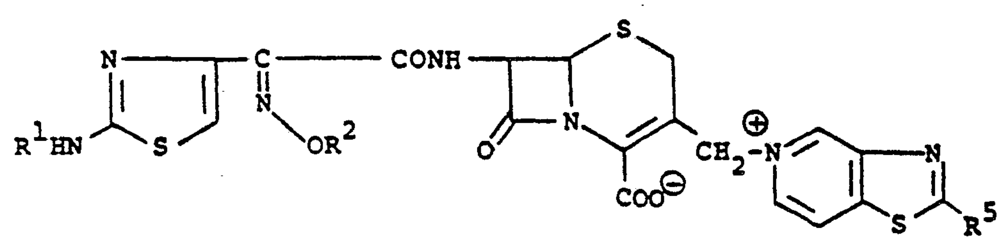

I

worin

$R^1$ Wasserstoff oder eine herkömmliche Aminoschutzgruppe ist;

$R^2$ eine geradkettige oder verzweigte Alkylgruppe mit 1 bis 4 Kohlenstoffatomen, Allyl, 2-Butenyl, 3-Butenyl oder eine Gruppe der Formel:

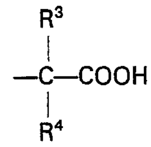

ist, worin

$R^3$ oder $R^4$ jeweils Wasserstoff, Methyl oder Ethyl bedeuten, oder $R^3$ und $R^4$ beide zusammen mit dem Kohlenstoffatom, an das sie gebunden sind, einen Cycloalkylidenring mit 3 bis 5 Kohlenstoffatomen bilden können, und

$R^5$ Wasserstoff, eine Aminogruppe, $C_1$—$C_6$-Alkylgruppe, $C_1$—$C_6$-Alkoxygruppe oder $C_1$—$C_6$-Alkylthiogruppe ist,

oder ein nicht-toxisches pharmazeutisch veträgliches Salz, ein pysiologisch hydrolysierbarer Ester oder Solvat davon.

2. Verbindungen nach Anspruch 1, worin $R^1$ Wasserstoff ist; $R^2$ Methyl, Ethyl, Allyl, 2-Carboxyprop-2-yl, 1-Carboxycycloprop-1-yliden oder 1-Carboxycyclobut-1-yliden ist; und $R^5$ Wasserstoff, Amino, Methyl oder Methylthio ist, oder ein nicht-toxisches pharmazeutisch verträgliches Salz, ein pysiologisch hydrolysierbarer Ester oder Solvat davon.

3. Verbindungen nach Anspruch 1, nämlich:

7-[(Z)-2-(2-Aminothiazol-4-yl)-2-methoxyiminoacetamido]-3-{(5-thiazolo[4,5-c]pyridinio)methyl}-3-cephem-4-carboxylat;

7-[(Z)-2-(2-Aminothiazol-4-yl)-2-ethoxyiminoacetamido]-3-{(5-thiazolo[4,5-c]pyridinio)methyl}-3-cephem-4-carboxylat;

7-[(Z)-2-(2-Allyloxyimino-2-(2-aminothiazol-4-yl)-acetamido]-3-{(5-thiazolo[4,5-c]pyridinio)methyl}-3-cephem-4-carboxylat;

7-[(Z)-2-(2-Aminothiazol-4-yl)-2-(2-carboxyprop-2-oxyimino)-acetamido]-3-{(5-thiazolo[4,5-c]-pyridinio)methyl}-3-cephem-4-carboxylat;

7-[(Z)-2-(2-Aminothiazol-4-yl)-2-(1-carboxycyclobut-1-oxyimino)-acetamido]-3-{(5-thiazolo[4,5-c]-pyridinio)methyl}-3-cephem-4-carboxylat;

7-[(Z)-2-(2-Aminothiazol-4-yl)-2-methoxyiminoacetamido]-3-[{5-(2-aminothiazolo[4,5-c]pyridinio)}-methyl]-3-cephem-4-carboxylat;

7-[(Z)-2-(2-Aminothiazol-4-yl)-2-ethoxyiminoacetamido]-3-[{5-(2-aminothiazolo[4,5-c]pyridinio)}-methyl]-3-cephem-4-carboxylat;

7-[(Z)-2-(2-Aminothiazol-4-yl)-2-(2-carboxyprop-2-oxyimino)-acetamido]-3-[{5-(2-aminothiazolo[4,5-c]-pyridinio)}methyl]-3-cephem-4-carboxylat;

7-[(Z)-2-(2-Aminothiazol-4-yl)-2-methoxyiminoacetamido-3-[{5-(2-methylthiothiazolo[4,5-c]pyridinio)}-methyl-3-cephem-4-carboxylat; und

7-[(Z)-2-(2-Aminothiazol-4-yl)-2-methoxyiminoacetamido-3-[{5-(2-methylthiazolo[4,5-c]pyridinio)}-methyl]-3-cephem-4-carboxylat

oder ein nicht-toxisches pharmazeutisch verträgliches Salz, ein pysiologisch hydrolysierbarer Ester oder ein Solvat davon.

4. Pharmazeutisches Mittel, enthaltend eine wirksame Menge mindestens einer Verbindung nach den Ansprüchen 1 bis 3 und einen inerten pharmazeutischen Träger.

5. Mittel nach Anspruch 4, wobei die Verbindung nach den Ansprüchen 1 bis 3 ausgewählt ist unter:

a) 7-[(Z)-2-(2-Aminothiazol-4-yl)-2-methoxyimino-acetamido]-3-{(5-thiazolo[4,5-c]pyridinio)methyl}-3-cephem-4-carboxylat,

b) 7-[(Z)-2-(2-Aminothiazol-4-yl)-2-(2-carboxyprop-2-oxyimino)acetamido]-3-{(5-thiazolo[4,5-c]-pyridinio)methyl}-3-cephem-4-carboxylat,

c) 7-[(Z)-2-(2-Aminothiazol-4-yl)-2-ethoxyiminoacetamido]-3-{(5-thiazolo[4,5-c]pyridinio)methyl}-3-cephem-4-carboxylat,

d) 7-[(Z)-2-(2-Aminothiazol-4-yl)-2-(1-carboxycyclobut-1-oxyimino)acetamido]-3-{(5-thiazolo[4,5-c]-pyridinio)methyl}-3-cephem-4-carboxylat,

e) 7-[(Z)-2-(2-Aminothiazol-4-yl)-2-methoxyiminoacetamido]-3-[{5-(2-aminothiazolo[4,5-c]pyridinio)}-methyl]-3-cephem-4-carboxylat,

f) 7-[(Z)-2-(2-Aminothiazol-4-yl)-2-methoxyiminoacetamido]-3-[{5-(2-methylthiazolo[4,5-c]pyridinio)}-methyl]-3-cephem-4-carboxylat,

und den nicht-toxischen pharmazeutisch verträglichen Salzen, physiologisch hydrolysierbaren Estern und Solvaten dieser Verbindungen.

6. Antibakterielles Mittel in Einheitsdosisform, enthaltend 50 bis 1500 mg mindestens einer Verbindung nach den Ansprüchen 1 bis 3 und einen inerten pharmazeutischen Träger.

7. Mittel nach Anspruch 6, wobei die Verbindung nach den Ansprüchen 1 bis 3 ausgewählt ist unter den in Anspruch 5 aufgeführten Verbindungen.

8. Verfahren zur Herstellung der Verbindungen nach den Ansprüchen 1 bis 3, dadurch gekennzeichnet, daß man eine Verbindung der allgemeinen Formel II:

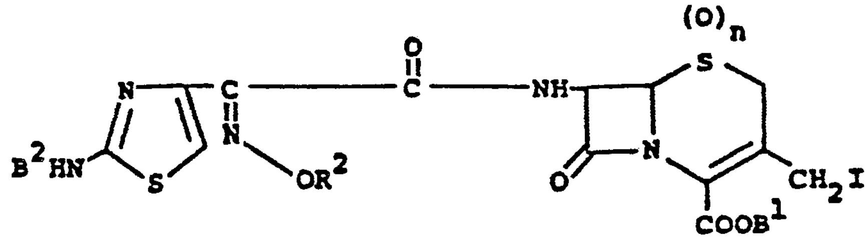

II

worin

$R^2$ wie in Anspruch 1 angegeben ist;

$B^1$ eine herkömmliche Carboxylschutzgruppe ist;

$B^2$ eine herkömmliche Aminoschutzgruppe ist, und

n für 0 oder 1 steht,

mit einem Thiazolo-[4,5-c]pyridin der Formel III:

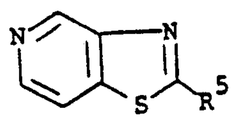

III

worin $R^5$ wie in Anspruch 1 angegeben ist, umsetzt, um eine Verbindung der Formel IV:

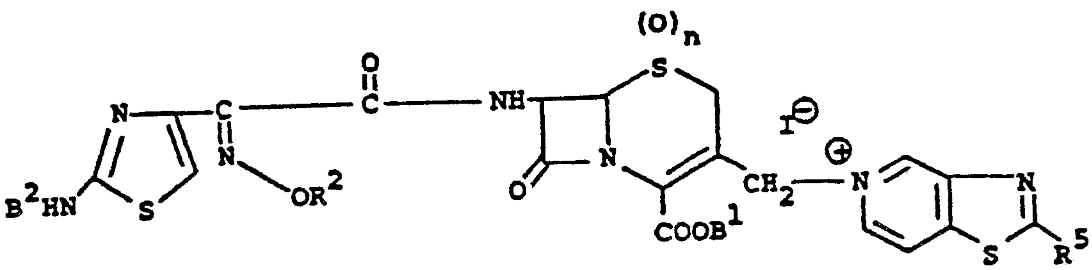

IV

zu erhalten worin

$R^2$, $R^5$, $B^1$ und $B^2$ und n wie oben definiert sind, und wobei man, wenn n für 1 steht, das Sulfoxid auf herkömmliche Weise reduziert und anschließend alle Schutzgruppen auf herkömmliche Weise entfernt, um Verbindungen der Formel I zu erhalten, worin $R^1$ Wasserstoff ist, ode die Schutzgruppe $B^1$ entfernt, um Verbindungen der Formel I zu erhalten, worin $R^1$ eine herkömmliche Aminoschutzgruppe ist, und/oder gewünschtenfalls die Verbindungen der Formel I in deren nicht-toxische pharmazeutisch verträgliche Salze, physiologisch hydrolysierbare Ester oder Solvate überführt.

9. Verfahren zur Herstellung der Verbindungen nach den Ansprüchen 1 bis 3, dadurch gekennzeichnet, daß man eine Verbindung der Formel V:

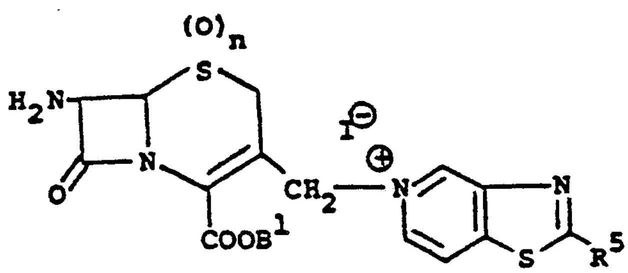

V

oder ein N-Silylderivat davon, worin $B^1$ Wasserstoff oder eine herkömmliche Carboxylschutzgruppe ist, n für 0 oder 1 steht, und $R^5$ wie in Anspruch 1 angegeben ist, mit einem acylierenden Derivat einer Säure der Formel VI:

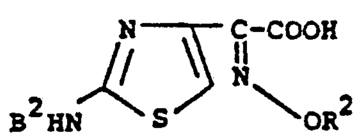

VI

acyliert,

worin

$R^2$ wie in Anspruch 1 angegeben ist, und jede Carboxylgruppe in $R^2$ durch eine herkömmliche Schutzgruppe $B^3$ geschützt ist, und

$B^2$ eine herkömmliche Aminoschutzgruppe ist, um eine Verbindung der Formel VIII:

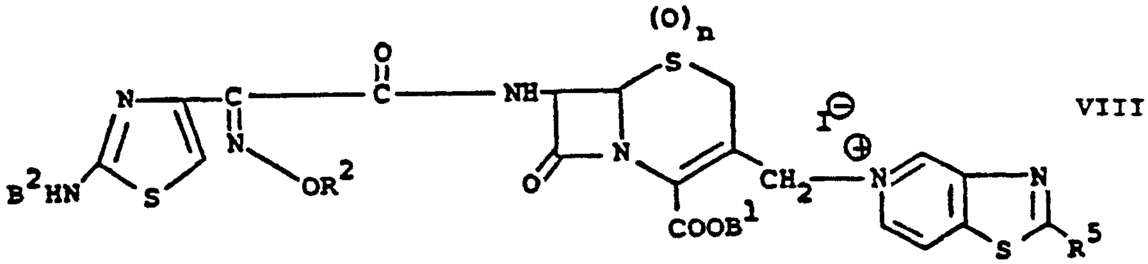

VIII

zu erhalten, worin

$R^2$, $R^5$, $B^1$, $B^2$ und n wie oben angegeben sind, und wobei man, wenn n für 1 steht, das Sulfoxid auf herkömmliche Weise reduziert und anschließend alle Schutzgruppen entfernt, um Verbindungen der Formel I herzustellen, worin $R^1$ Wasserstoff ist, oder die Schutzgruppen $B^1$ und $B^3$ entfernt, um Verbindungen der Formel I zu erhalten, worin $R^1$ eine herkömmliche Aminoschutzgruppe ist, und/oder gewünschtenfalls die Verbindungen der Formel I in deren nicht-toxische pharmazeutisch verträgliche Salze, physiologisch hydrolysierbare Ester oder Solvate überführt.

**Patentansprüche für den Vertragsstaat: AT**

1. Verfahren zur Herstellung der Verbindungen der allgemeinen Formel I:

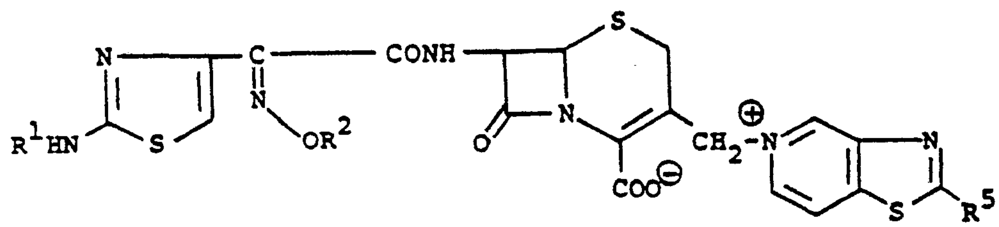

I

worin

$R^1$ Wasserstoff oder eine herkömmliche Aminoschutzgruppe ist;

$R^2$ eine geradkettige oder verzweigte Alkylgruppe mit 1 bis 4 Kohlenstoffatomen, Allyl, 2-Butenyl, 3-Butenyl oder eine Gruppe der Formel:

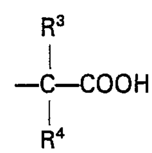

ist, worin

$R^3$ oder $R^4$ jeweils Wasserstoff, Methyl oder Ethyl bedeuten, oder $R^3$ und $R^4$ beide zusammen mit dem Kohlenstoffatom, an das sie gebunden sind, einen Cycloalkylidenring mit 3 bis 5 Kohlenstoffatomen bilden können, und

$R^5$ Wasserstoff, eine Aminogruppe, $C_1$—$C_6$-Alkylgruppe, $C_1$—$C_6$-Alkoxygruppe oder $C_1$—$C_6$-Alkylthiogruppe ist,

oder ein nicht-toxisches pharmazeutisch veträgliches Salz, ein pysiologisch hydrolysierbarer Ester oder Solvat davon, dadurch gekennzeichnet, daß man

A) eine Verbindung der allgemeinen Formel II:

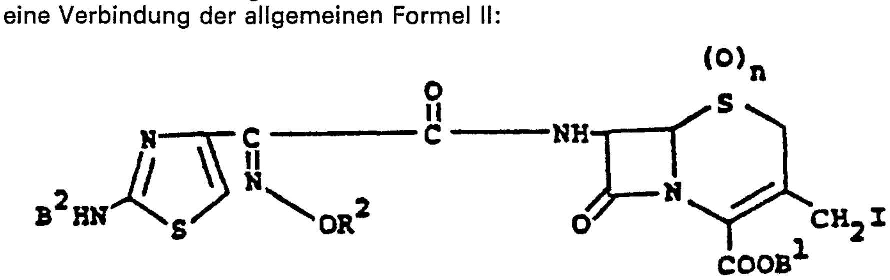

II

worin

$R^2$ wie oben angegeben ist;

$B^1$ eine herkömmliche Carboxylschutzgruppe ist;

$B^2$ eine herkömmliche Aminoschutzgruppe ist, und

n für 0 oder 1 steht,

mit einem Thiazolo-[4,5-c]pyridin der Formel III:

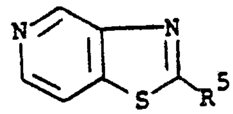

III

worin $R^5$ wie oben angegeben ist, umsetzt, um eine Verbindung der Formel IV:

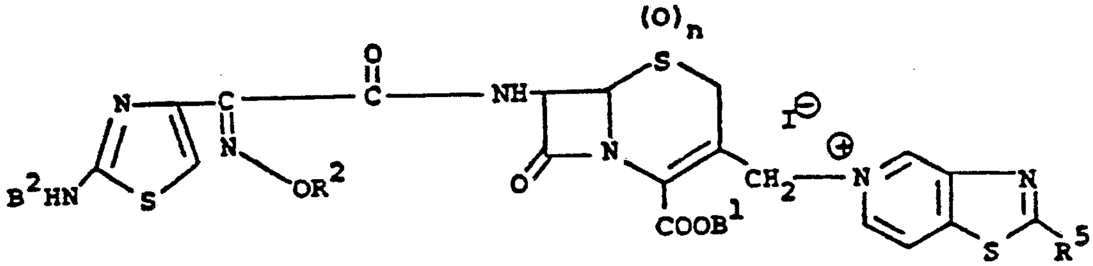

IV

zu erhalten worin

$R^2$, $R^5$, $B^1$ und $B^2$ und n wie oben definiert sind, und wobei man, wenn n für 1 steht, das Sulfoxid auf herkömmliche Weise reduziert und anschließend all Schutzgruppen auf herkömmliche Weise entfernt, um Vebrindungen der Formel I zu erhalten, worin $R^1$ Wasserstoff ist, oder die Schutzgruppe $B^1$ entfernt, um Verbindungen der Formel I zu erhalten, worin $R^1$ eine herkömmliche Aminoschutzgruppe ist, und/oder gewünschtenfalls die Verbindungen der Formel I in deren nicht-toxische pharmazeutisch verträgliche Salze, physiologisch hydrolysierbare Ester oder Solvate überführt, oder

B) eine Verbindung der Formel V:

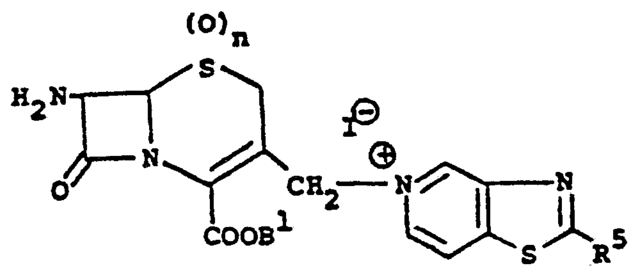

V

oder ein N-Silylderivat davon, worin $B^1$ Wasserstoff oder eine herkömmliche Carboxylschutzgruppe ist, n für 0 oder 1 steht, und $R^5$ wie oben angegeben ist, mit einem acylierenden Derivat einer Säure der Formel VI:

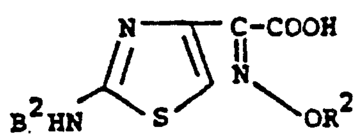

VI

worin

$R^2$ wie oben angegeben ist, und jede Carboxylgruppe in $R^2$ durch eine herkömmliche Schutzgruppe $B^3$ geschützt ist, und

$B^2$ eine herkömmliche Aminoschutzgruppe ist, acyliert, um eine Verbindung der Formel VIII:

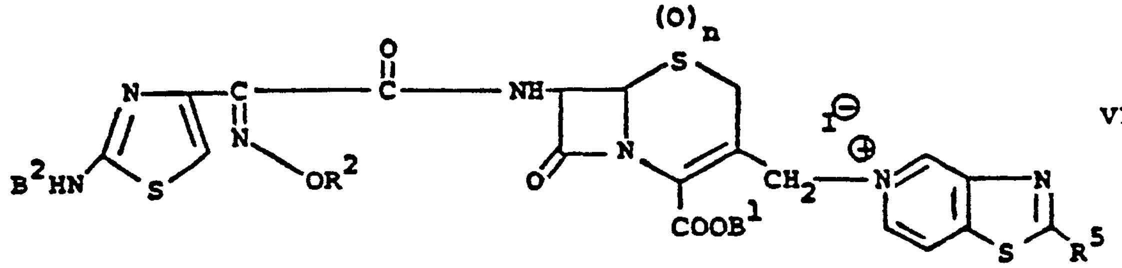

VIII

zu erhalten, worin

$R^2$, $R^5$, $B^1$, $B^2$ und n wie oben angegeben sind, und wobei man, wenn n für 1 steht, das Sulfoxid auf herkömmliche Weise reduziert und anschließend alle Schutzgruppen entfernt, um Verbindungen der Formel I herzustellen, worin $R^1$ Wasserstoff ist, oder die Schutzgruppen $B^1$ und $B^3$ entfernt, um Verbindungen der Formel I zu erhalten, worin $R^1$ eine herkömmliche Aminoschutzgruppe ist, und/oder gewünschtenfalls die Verbindungen der Formel I in deren nicht-toxische pharmazeutisch verträgliche Salze, physiologisch hydrolysierbare Ester oder Solvate überführt.

2. Verfahren nach Anspruch 1, dadurch gekennzeichnet, daß man Verbindungen der Formel I, worin $R^1$ Wasserstoff ist; $R^2$ Methyl, Ethyl, Allyl, 2-Carboxyprop-2-yl, 1-Carboxycycloprop-1-yliden oder 1-Carboxycyclobut-1-yliden ist; und $R^5$ Wasserstoff, Amino, Methyl oder Methylthio ist, oder ein nicht-toxisches pharmazeutisch verträgliches Salz, einen physiologisch hydrolysierbaren Ester oder ein Solvat davon erhalt.

3. Verfahren nach Anspruch 1, dadurch gekennzeichnet, daß man die folgenden Verbindungen erhält:

7-[(Z)-2-(2-Aminothiazol-4-yl)-2-methoxyiminoacetamido]-3-{(5-thiazolo[4,5-c]pyridinio)methyl}-3-cephem-4-carboxylat;

7-[(Z)-2-(2-Aminothiazol-4-yl)-2-ethoxyiminoacetamido]-3-{(5-thiazolo[4,5-c]pyridinio)methyl}-3-cephem-4-carboxylat;

7-[(Z)-2-(2-Allyloxyimino-2-(2-aminothiazol-4-yl)-acetamido-3-{(5-thiazolo[4,5-c]pyridinio)methyl}-3-cephem-4-carboxylat;

7-[(Z)-2-(2-Aminothiazol-4-yl)-2-(2-carboxyprop-2-oxyimino)-acetamido]-3-{(5-thiazolo[4,5-c]-pyridinio)methyl}-3-cephem-4-carboxylat;

7-[(Z)-2-(2-Aminothiazol-4-yl)-2-(1-carboxycyclobut-1-oxyimino)-acetamido]-3-{(5-thiazolo[4,5-c]-pyridinio)methyl}-3-cephem-4-carboxylat;

7-[(Z)-2-(2-Aminothiazol-4-yl)-2-methoxyiminoacetamido]-3-[{5-(2-aminothiazolo[4,5-c]pyridinio)}-methyl]-3-cephem-4-carboxylat;

7-[(Z)-2-(2-Aminothiazol-4-yl)-2-ethoxyiminoacetamido]-3-[{5-(2-aminothiazolo[4,5-c]pyridinio)}-methyl]-3-cephem-4-carboxylat;

7-[(Z)-2-(2-Aminothiazol-4-yl)-2-(2-carboxyprop-2-oxyimino)-acetamido]-3-[{5-(2-aminothiazolo[4,5-c]-pyridinio)}methyl]-3-cephem-4-carboxylat;

7-[(Z)-2-(2-Aminothiazol-4-yl)-2-methoxyiminoacetamido-3-[{5-(2-methylthiothiazolo[4,5-c]pyridinio)}-methyl-3-cephem-4-carboxylat; und

7-[(Z)-2-(2-Aminothiazol-4-yl)-2-methoxyiminoacetamido-3-[{5-(2-methylthiazolo[4,5-c]pyridinio)}-methyl]-3-cephem-4-carboxylat.

oder ein nicht-toxisches pharmazeutisch verträgliches Salz, einen pysiologisch hydrolysierbaren Ester oder ein Solvat davon.

4. Verfahren zur Herstellung eines pharmazeutischen Mittels, dadurch gekennzeichnet, daß man eine pharmazeutisch wirksame Menge mindestens einer der Verbindungen nach den Ansprüchen 1 bis 3 und einem inerten pharmazeutischen Träger inkorporiert.

5. Verfahren nach Anspruch 4, wobei die Verbindung nach den Ansprüchen 1 bis 3 ausgewählt ist unter:

a) 7-[(Z)-2-(2-Aminothiazol-4-yl)-2-methoxyimino-acetamido]-3-{(5-thiazolo[4,5-c]pyridinio)methyl}-3-cephem-4-carboxylat,

b) 7-[(Z)-2-(2-Aminothiazol-4-yl)-2-(2-carboxyprop-2-oxyimino)acetamido]-3-{(5-thiazolo[4,5-c]-pyridinio)methyl}-3-cephem-4-carboxylat,

c) 7-[(Z)-2-(2-Aminothiazol-4-yl)-2-ethoxyiminoacetamido]-3-{(5-thiazolo[4,5-c]pyridinio)methyl}-3-cephem-4-carboxylat,

d) 7-[(Z)-2-(2-Aminothiazol-4-yl)-2-(1-carboxycyclobut-1-oxyimino)acetamido]-3-{(5-thiazolo[4,5-c]-pyridinio)methyl}-3-cephem-4-carboxylat,

e) 7-[(Z)-2-(2-Aminothiazol-4-yl)-2-methoxyiminoacetamido]-3-[{5-(2-aminothiazolo[4,5-c]pyridinio)}-methyl]-3-cephem-4-carboxylat,

f) 7-[(Z)-2-(2-Aminothiazol-4-yl)-2-methoxyiminoacetamido]-3-[{5-(2-methylthiazolo[4,5-c]pyridinio)}-methyl-3-cephem-4-carboxylat,

und den nicht-toxischen pharmazeutisch verträglichen Salzen, physiologisch hydrolysierbaren Estern und Solvaten dieser Verbindungen.

6. Verfahren zur Herstellung eines antibakteriellen Mittels in Einheitsdosisform, dadurch gekennzeichnet, daß man 50 mg bis 1500 mg mindestens einer Verbindung nach den Ansprüchen 1 bis 3 einem inerten pharmazeutischen Träger inkorporiert.

7. Verfahren nach Anspruch 6, wobei die Verbindung nach den Ansprüchen 1 bis 3 ausgewählt ist unter den in Anspruch 5 aufgeführten Verbindungen.

**Revendications pour les Etats contractants: BE CH DE FR GB IT LI LU NL SE**

1. Composés de formule générale I:

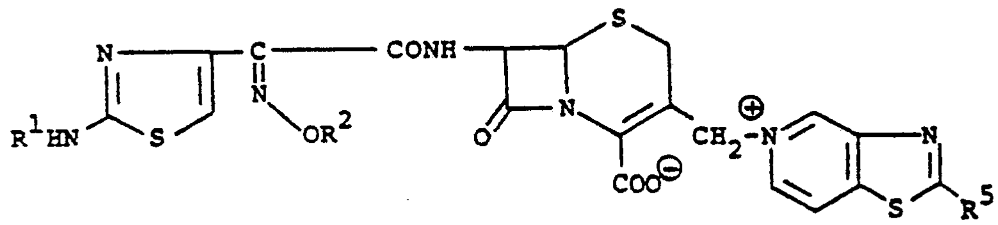

I

dans laquelle:

$R^1$ est un atome d'hydrogène ou un groupe protecteur d'amine classique;

$R^2$ est un radical alkyle à chaîne linéaire ou ramifiée contenant 1 à 4 atomes de carbone, allyle, 2-buténvle, 3-buténvle ou un groupe de formule:

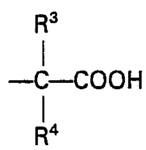

dans laquelle $R^3$ et $R^4$, indépendamment l'un de l'autre, consistent en un atome d'hydrogène ou un radical méthyle ou éthyle; ou $R^3$ et $R^4$ pris ensemble avec l'atome de carbone auquel ils sont liés forment un noyau cycloalkylidène contenant de 3 à 5 atomes de carbone;

$R^5$ est un atome d'hydrogène ou un groupe amino, $(C_1-C_6)$-alkyle, $(C_1-C_6)$-alkoxy ou $(C_1-C_6)$-alkylthio;

ou un sel non toxique pharmaceutiquement acceptable, un ester physiologiquement hydrolysable ou un solvate de ces composés.

2. Composés selon la revendication 1, dans lequel: $R^1$ est un atome d'hydrogène, $R^2$ est un radical méthyle, éthyle, allyle, 2-carboxyprop-2-yl, 1-carboxycycloprop-1-ylidène ou carboxycyclobut-1-ylidène, et $R^5$ est un atome d'hydrogène, un groupe amino, méthyle ou méthylthio; ou un sel non toxique pharmaceutiquement acceptable, un ester physiologiquement hydrolysable ou un solvate de ces composés.

3. Composés selon la revendication 1, respectivement:

7-[(Z)-2-(2-aminothiazol-4-yl)-2-méthoxyiminoacétamido]-3-{(5-thiazolo[4,5-c]pyridinio)méthyl}-3-céphem-4-carboxylate;

7-[(Z)-2-(2-aminothiazol-4-yl)-2-éthoxyiminoacétamido]-3-{(5-thiazolo[4,5-c]pyridinio)méthyl}-3-céphem-4-carboxylate;

7-[(Z)-2-(2-allyloxyimino)-2-(2-aminothiazol-4-yl)-acétamido]-3-{(5-thiazolo[4,5-c]pyridinio)méthyl}-3-céphem-4-carboxylate;

7-[(Z)-2-(2-aminothiazol-4-yl)-2-(2-carboxyprop-2-oxyimino)-acétamido]-3-{(5-thiazolo[4,5-c]pyridinio)-méthyl}-3-céphem-4-carboxylate;

7-[(Z)-2-(2-aminothiazol-4-yl)-2-(1-carboxycyclobut-1-oxyimino)-acétamido]-3-{(5-thiazolo[4,5-c]-pyridinio)méthyl}-3-céphem-4-carboxylate;

7-[(Z)-2-(2-aminothiazol-4-yl)-2-méthoxyiminoacétamido]-3-{[5-(2-aminothiazolo[4,5-c]pyridinio)]-méthyl}-3-céphem-4-carboxylate;

7-[(Z)-2-(2-aminothiazol-4-yl)-2-éthoxyiminoacétamido]-3-{[5-(2-aminothiazolo[4,5-c]pyridinio)]-méthyl}-3-céphem-4-carboxylate;

7-[(Z)-2-(2-aminothiazol-4-yl)-2-(2-carboxyprop-2-oxyimino)-acétamido]-3-{[5-(2-aminothiazolo[4,5-c]-pyridinio)]méthyl}-3-céphem-4-carboxylate;

7-[(Z)-2-(2-aminothiazol-4-yl)-2-méthoxyiminoacétamido]-3-{[5-(2-méthylthiothiazolo[4,5-c]pyridinio)]-méthyl}-3-céphem-4-carboxylate; et

7-[(Z)-2-(2-aminothiazol-4-yl)-2-méthoxyiminoacétamido]-3-{[5-(2-méthylthiazolo[4,5-c]pyridinio)]-méthyl}-3-céphem-4-carboxylate;

ou un sel non toxique pharmaceutiquement acceptable, un ester physiologiquement hydrolysable ou un solvate de ces composés.

4. Composition pharmaceutique comprenant une quantité efficace du point de vue pharmaceutique d'un moins un composé selon les revendicatons 1 à 3, ainsi qu'un support inerte pharmaceutiquement acceptable.

5. Composition selon la revendication 4, dans laquelle le composé selon les revendications 1 à 3 est choisi dans le groupe comprenant:

a) 7-[(Z)-2-(2-aminothiazol-4-yl)-2-méthoxyiminoacétamido]-3-{(5-thiazolo[4,5-c]pyridinio)méthyl}-3-céphem-4-carboxylate;

b) 7-[(Z)-2-(2-aminothiazol-4-yl)-2-(2-carboxyprop-2-oxyimino)-acétamido]-3-{(5-thiazolo[4,5-c]-pyridinio)méthyl}-3-céphem-4-carboxylate;

c) 7-[(Z)-2-(2-aminothiazol-4-yl)-2-éthoxyiminoacétamido]-3-{(5-thiazolo[4,5-c]pyridinio)méthyl}-3-céphem-4-carboxylate;

d) 7-[(Z)-2-(2-aminothiazol-4-yl)-2-(1-carboxycyclobut-1-oxyimino)-acétamido]-3-{(5-thiazolo[4,5-c]-pyridinio)méthyl}-3-céphem-4-carboxylate;

e) 7-[(Z)-2-(2-aminothiazol-4-yl)-2-méthoxyiminoacétamido]-3-{5-(2-aminothiazolo[4,5-c]pyridinio)-méthyl}-3-céphem-4-carboxylate;

f) 7-[(Z)-2-(2-aminothiazol-4-yl)-2-méthoxyiminoacétamido]-3-{5-(2-méthylthiazolo[4,5-c]pyridinio)-méthyl}-3-céphem-4-carboxylate;

ainsi que les sels non toxiques pharmaceutiquement acceptables, les esters physiologiquement hydrolysables ou les solvates de ces composés.

6. Une composition antibactérienne sous forme de dose unitaire, comprenant d'environ 50 à environ 1 500 mg d'au moins un composé selon les revendications 1 à 3 ainsi qu'un support pharmaceutiquement inerte.

7. Composition selon la revendication 6, dans laquelle le composé selon les revendications 1 à 3 est choisi dans le groupe tel que défini dans la revendication 5.

8. Procédé de fabrication des composés selon les revendications 1 à 3, caractérisé en ce qu'un composé de formule générale II:

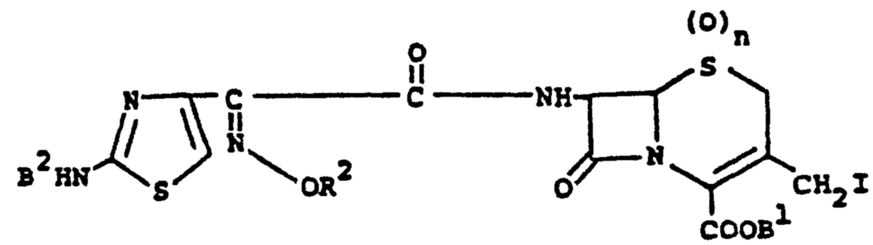

II

dans laquelle:

$R^2$ est tel que défini dans la revendication 1;

$B^1$ est un groupe protecteur de carboxyle classique;

$B^2$ est un groupe protecteur d'amine classique;

n est égal à 0 ou 1,

est amené à réagir avec un thiazolo[4,5-c]pyridine de formule III:

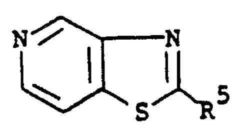

III

dans laquelle $R^5$ est tel que défini dans la revendication 1, pour produire un composé de formule IV:

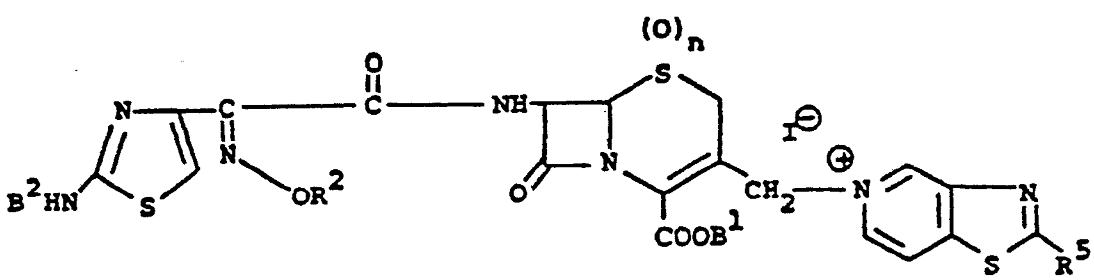

IV

dans laquelle

$R^2$, $R^5$, $B^1$, $B^2$ et n sont tels que définis ci-dessus,

et, si n est égal à 1, le sulfoxyde est réduit par des moyens conventionnels, et ultérieurement tous les groupes protecteurs sont éliminés par des moyens classiques pour conduire au composé de formule I, dans laquelle $R^1$ est un atome d'hydrogène ou le groupe protecteur $B^1$ est éliminé pour conduire au

composé de formule I dans laquelle $R^1$ est un groupe protecteur d'amine classique, et/ou le cas échéant, les composés de formule I sont convertis en leurs sels pharmaceutiquement acceptables non toxiques, esters physiologiquement hydrolysables et solvates dérivant de ces composés.

9. Procédé pour le fabrication des composés selon les revendicatons 1 à 3, caractérisé en ce qu'un composé de formule V:

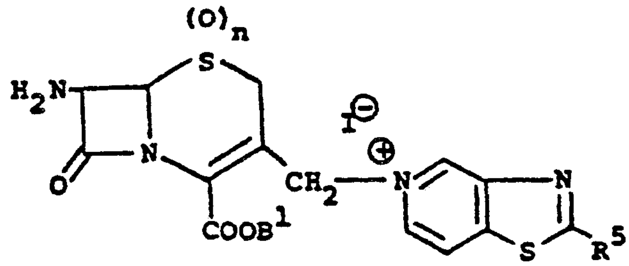

V

ou un composé N-silyle en dérivant, formule dans laquelle:

$B^1$ est un atome d'hydrogène ou un groupe protecteur de carboxyle classique;

n est égal à 0 ou 1; et

$R^5$ est tel que défini dans la revendication 1, est acylé par un dérivé acylant d'un acide de formule VI:

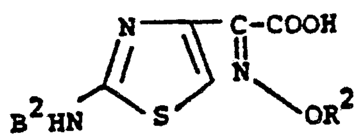

VI

dans laquelle:

$R^2$ est tel que défini dans la revendication 1, et tout groupe carboxyle dans lequel $R^2$ est protégé par un groupe protecteur classique $B_3$; et

$B^2$ est un groupe protecteur d'amine classique, pour produire un compose de formule VIII:

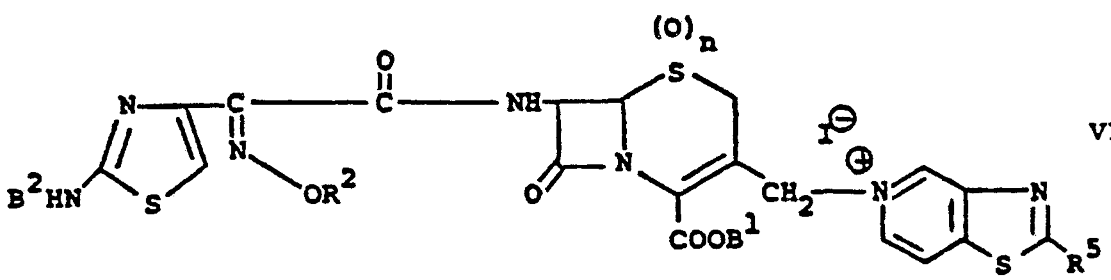

VIII

dans laquelle

$R^2$, $R^5$, $B^1$, $B^2$ et n sont tels que définis ci-dessus,

et, si n est égal à 1, le sulfoxyde est réduit par des moyens conventionnels, et ultérieurement tous les groupes protecteurs sont éliminés pour produire les composés de formule I dans laquelle $R^1$ est un atome d'hydrogéne ou les groupes protecteurs $B^1$ et $B^3$ sont éliminés pour conduire aux composés de formule I dans laquelle $R^1$ est un groupe protecteur d'amine classique et/ou, le cas échéant, les composés de formule I sont convertis en leurs sels pharmaceutiquement acceptables non toxiques, esters physiologiquement hydrolysables et solvates dérivant de ces composés.

**Revendications pour l'Etat contractant: AT**

1. Un procédé de fabrication des composés de formule générale I:

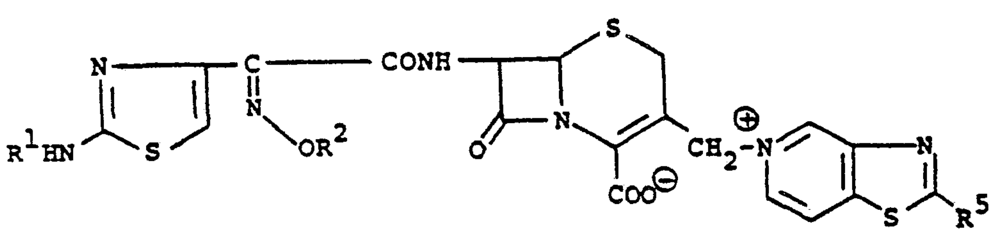

I

dans laquelle:

$R^1$ est un atome d'hydrogène ou un groupe protecteur d'amine classique;

$R^2$ est un radical alkyle à chaîne linéaire ou ramifiée contenant 1 à 4 atomes de carbone, allyle, 2-buténvle, 3-buténvle ou un groupe de formule:

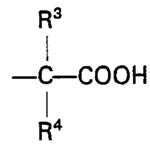

dans laquelle $R^3$ et $R^4$, indépendamment l'un de l'autre, consistent en un atome d'hydrogène ou un radical méthyle ou éthyle; ou $R^3$ et $R^4$ pris ensemble avec l'atome de carbone auquel ils sont liés forment un noyau cycloalkylidène contenant de 3 à 5 atomes de carbone;

$R^5$ est un atome d'hydrogène ou un groupe amino, $(C_1$—$C_6)$-alkyle, $(C_1$—$C_6)$-alkoxy ou $(C_1$—$C_6)$-alkylthio;

ou un sel non toxique pharmaceutiquement acceptable, un ester physiologiquement hydrolysable ou un solvate de ces composés, caractérisé en ce que:

A) on fait réagir un composé de formule générale II:

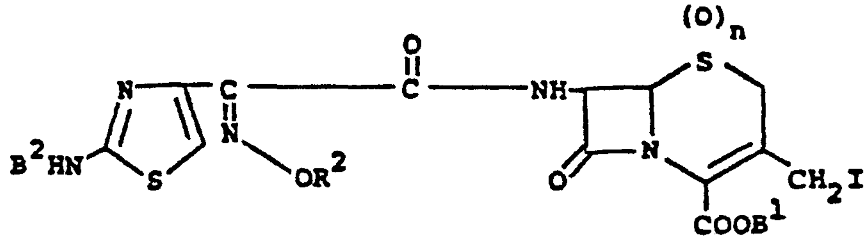

II

dans laquelle:

$R^2$ est tel que défini ci-dessus;

$B^1$ est un groupe protecteur de carboxyle classique;

$B^2$ est un groupe protecteur d'amine classique;

n est égal à 0 ou 1,

avec un thiazolo[4,5-c]pyridine de formule III:

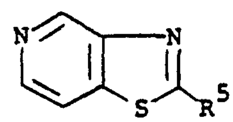

III

dans laquelle $R^5$ est tel que défini ci-dessus, pour produire un composé de formule IV:

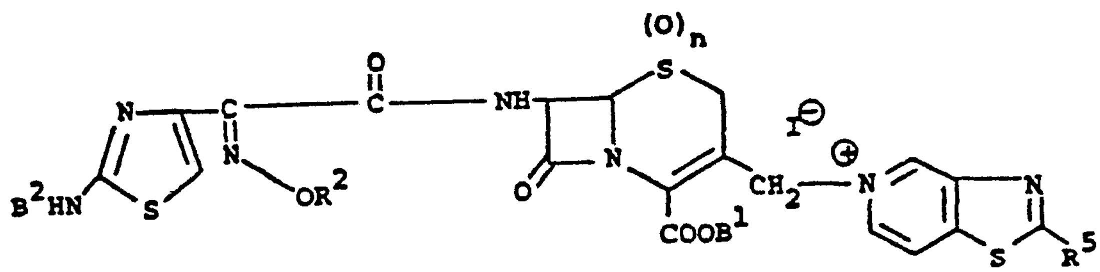

IV

dans laquelle

$R^2$, $R^5$, $B^1$, $B^2$ et n sont tels que définis ci-dessus,

et, si n est égal à 1, réduit le sulfoxyde par des moyens conventionnels, et ultérieurement élimine tous les groupes protecteurs par des moyens classiques pour conduire au composé de formule I, dans laquelle $R^2$ est un atome d'hydrogène ou élimine le groupe protecteur $B^1$ pour conduire au composé de formule I dans laquelle $R^1$ est un groupe protecteur d'amine classique, et ou le cas échéant, convertit les composés de formule I en leurs sels pharmaceutiquement acceptables non toxiques, esters physiologiquement hydrolysables et solvates dérivant de ces composés, ou

B) acyle un composé de formule V:

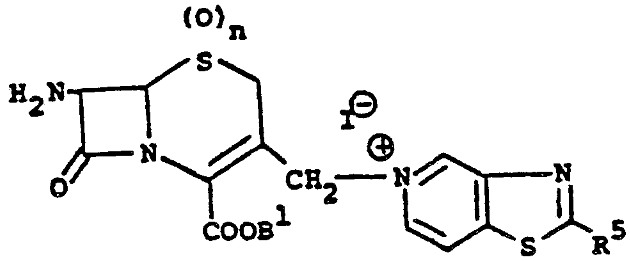

V

ou un composé N-silyle en dérivant, dans laquelle:

$B^1$ est un atome d'hydrogène ou un groupe protecteur de carboxyle classique;

n est égal à 0 ou 1; et

$R^5$ est tel que défini ci-dessus,

par un dérivé acylant d'un acide de formule VI:

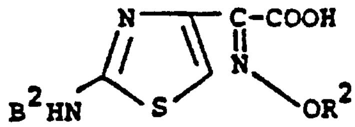

VI

dans laquelle:

$R^2$ est tel que défini ci-dessus, et tout groupe carboxyle $R^2$ est protégé par un groupe protecteur classique $B_3$; et

$B^2$ est un groupe protecteur d'amine classique, pour produire un composé de formule VIII:

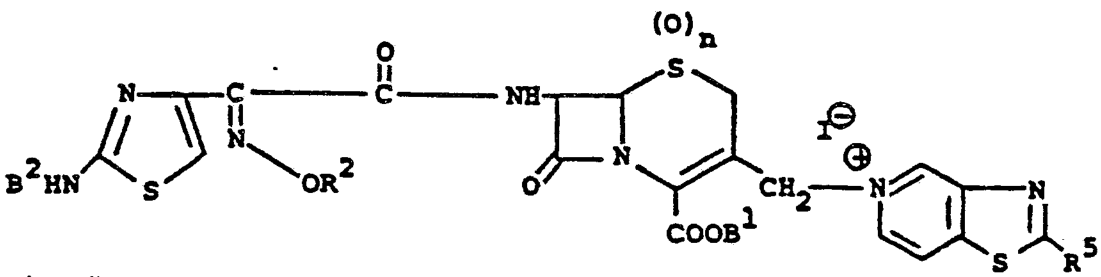

'VIII

dans laquelle

$R^2$, $R^5$, $B^1$, $B^2$ et n sont tels que définis ci-dessus,

et, si n est égal à 1, réduit le sulfoxyde par des moyens conventionnels, et ultérieurement élimine tous les groupes protecteurs pour produire les composés de formule I dans laquelle $R^1$ est un atome d'hydrogène ou élimine les groupes protecteurs $B^1$ et $B^3$ pour conduire aux composés de formule I dans laquelle $R^1$ est un groupe protecteur d'amine classique

et/ou, le cas échéant, convertot les composés de formule I en leurs sels pharmaceutiquement acceptables non toxiques, esters physiologiquement hydrolysables et solvates dérivant de ces composés.

2. Le procédé selon la revendication 1, caractérisé en ce qu'il conduit à l'obtention des composés de formule I, dans laquelle:

$R^1$ est un atome d'hydrogène,

$R^2$ est un radical méthyle, éthyle, allyle, 2-carboxyprop-2-yl, 1-carboxycycloprop-1-ylidène ou carboxycyclobut-1-ylidène, et

$R^5$ est un atome d'hydrogène, un groupe amino, méthyl ou méthylthio;

ou à un sel non toxique pharmaceutiquement acceptable, un ester physiologiquement hydrolysable ou un solvate de ces composés.

3. Le procédé selon la revendication 1, caractérisé en ce qu'il conduit à l'obtention des composés suivants:

7-[(Z)-2-(2-aminothiazol-4-yl)-2-méthoxyiminoacétamido]-3-{(5-thiazolo[4,5-c]pyridinio)méthyl}-3-céphem-4-carboxylate;

7-[(Z)-2-(2-aminothiazol-4-yl)-2-éthoxyiminoacétamido]-3-{(5-thiazolo[4,5-c]pyridinio)méthyl}-3-céphem-4-carboxylate;

7-[(Z)-2-(2-allyloxyimino)-2-(2-aminothiazol-4-yl)-acétamido]-3-{(5-thiazolo[4,5-c]pyridinio)méthyl}-3-céphem-4-carboxylate;

7-[(Z)-2-(2-aminothiazol-4-yl)-2-(2-carboxyprop-2-oxyimino)-acétamido]-3-{(5-thiazolo[4,5-c]pyridinio)-méthyl}-3-céphem-4-carboxylate;

7-[(Z)-2-(2-aminothiazol-4-yl)-2-(1-carboxycyclobut-1-oxyimino)-acétamido]-3-{(5-thiazolo[4,5-c]-pyridinio)méthyl}-3-céphem-4-carboxylate;

7-[(Z)-2-(2-aminothiazol-4-yl)-2-méthoxyiminoacétamido]-3-{[5-(2-aminothiazolo[4,5-c]pyridinio)]-méthyl}-3-céphem-4-carboxylate;

7-[(Z)-2-(2-aminothiazol-4-yl)-2-éthoxyiminoacétamido]-3-{[5-(2-aminothiazolo[4,5-c]pyridinio)]-méthyl}-3-céphem-4-carboxylate;

7-[(Z)-2-(2-aminothiazol-4-yl)-2-(2-carboxyprop-2-oxyimino)-acétamido]-3-{[5-(2-aminothiazolo[4,5-c]-pyridinio)]méthyl}-3-céphem-4-carboxylate;

7-[(Z)-2-(2-aminothiazol-4-yl)-2-méthoxyiminoacétamido]-3-{[5-(2-méthylthiothiazolo[4,5-c]pyridinio)]-méthyl}-3-céphem-4-carboxylate; et

7-[(Z)-2-(2-aminothiazol-4-yl)-2-méthoxyiminoacétamido]-3-{[5-(2-méthylthiazolo[4,5-c]pyridinio)]-méthyl}-3-céphem-4-carboxylate;

ou à un sel non toxique pharmaceutiquement acceptable, un ester physiologiquement hydrolysable ou un solvate de ces composés.

4. Un procédé de préparation d'une composition pharmaceutique, caractérisé en ce qu'un quantité efficace du point de vue pharmaceutique d'au moins un composé obtenu selon une des revendications 1 à 3 est incorporé dans un support inerte pharmaceutiquement acceptable.

5. Le procédé selon la revendication 4, dans laquelle le composé selon des revendications 1 à 3 est choisi dans le groupe comprenant:

a) 7-[(Z)-2-(2-aminothiazol-4-yl)-2-méthoxyiminoacétamido]-3-{(5-thiazolo[4,5-c]pyridinio)méthyl}-3-céphem-4-carboxylate;

b) 7-[(Z)-2-(2-aminothiazol-4-yl)-2-(2-carboxyprop-2-oxyimino)-acétamido]-3-{(5-thiazolo[4,5-c]-pyridinio)méthyl}-3-céphem-4-carboxylate;

c) 7-[(Z)-2-(2-aminothiazol-4-yl)-2-éthoxyiminoacétamido]-3-{(5-thiazolo[4,5-c]pyridinio)méthyl}-3-céphem-4-carboxylate;

d) 7-[(Z)-2-(2-aminothiazol-4-yl)-2-(1-carboxycyclobut-1-oxyimino)-acétamido]-3-{(5-thiazolo[4,5-c]-pyridinio)méthyl}-3-céphem-4-carboxylate;

e) 7-[(Z)-2-(2-aminothiazol-4-yl)-2-méthoxyiminoacétamido]-3-{5-(2-aminothiazolo[4,5-c]pyridinio)-méthyl}-3-céphem-4-carboxylate;

f) 7-[(Z)-2-(2-aminothiazol-4-yl)-2-méthoxyiminoacétamido]-3-{5-(2-méthylthiazolo[4,5-c]pyridinio)-méthyl}-3-céphem-4-carboxylate;

ainsi que des sels non toxiques pharmaceutiquement acceptables, des esters physiologiquement hydrolysables ou des solvates de ces composés.

6. Un procédé de préparation d'une composition antibactérienne sous forme de dose unitaire, caractérisé en ce que l'on incorpore d'environ 50 à environ 1 500 mg d'au moins un composé obtenu selon les revendications 1 à 3 dans un support pharmaceutiquement inerte.

7. Le procédé selon la revendication 6, dans lequel le composé selon les revendications 1 à 3 est choisi dans le groupe ainsi que decrit dans la revendication 5.